# EUROPÄISCHE PATENTSCHRIFT

(11) **EP 2 373 647 B1**
(45) Veröffentlichungstag und Bekanntmachung des Hinweises auf die Patenterteilung: **31.10.2012**
(21) Anmeldenummer: 09768339.5
(22) Anmeldetag: 10.12.2009
(51) Int. Cl.: C07D 413/10, C07D 413/14, C07D 417/10, C07D 417/14, A61K 31/501, A61P 35/00

(54) **PYRIDAZINONDERIVATE**
PYRIDAZINONE DERIVATIVES
DÉRIVÉS DE PYRIDAZINONE

(30) Priorität: 08.01.2009 DE 102009004061
(43) Veröffentlichungstag der Anmeldung: 12.10.2011
(73) Patentinhaber: Merck Patent GmbH, 64293 Darmstadt (DE)
(72) Erfinder: DORSCH, Dieter, 64372 Ober-Ramstadt (DE); SCHADT, Oliver, 63517 Rodenbach (DE); STIEBER, Frank, 69121 Heidelberg (DE); BLAUKAT, Andree, 69198 Schriesheim (DE)
(86) Internationale Anmeldenummer: PCT/EP2009/008839
(87) Internationale Veröffentlichungsnummer: WO 2010/078909

(56) Entgegenhaltungen:
- WO-A-2008/017361
- WO-A-2009/083076

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Offenbarung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten. Die vorliegende Erfindung betrifft insbesondere Verbindungen und die Verwendung von Verbindungen, bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.
Ein anderer Met-Kinase-Inhibitor zur Krebstherapie ist von J.G. Christensen et al. in Cancer Res. 2003, 63(21), 7345-55 beschrieben.
Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung Verbindungen der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Die vorliegende Erfindung Offenbarung sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinische Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Dihydropyridazinone zur Krebsbekämpfung sind in WO 03/037349 A1 beschrieben.
Andere Pyridazine zur Behandlung von Krankheiten des Immunsystems, ischämischer und entzündlicher Erkrankungen kennt man aus EP 1 043 317 A1 und EP 1 061 077 A1.
In EP 0 738 716 A2 und EP 0 711 759 B1 sind andere Dihydropyridazinone und Pyridazinone als Fungizide und Insektizide beschrieben. Andere Pyridazinone sind als cardiotonische Agenzien in US 4,397,854 beschrieben.

In JP 57-95964 sind andere Pyridazinone offenbart.
Andere Pyridazinonderivate sind als Met-Kinase-inhibitoren in WO 2008/017361 und WO 2007/065518 beschrieben.

### ZUSAMMENFASSUNG DER ERFINDUNG

Die Erfindung betrifft Verbindungen der Formel I 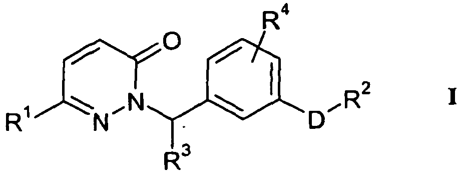 worin
- D: einen fünfgliedrigen ungesättigten oder aromatischen Heterocyclus mit 1 bis 3 N-, O- und/oder S-Atomen, der unsubstituiert oder ein-, zwei- oder dreifach durch Hal und/oder A substituiert sein kann,
- R¹: Ar, Het, A, OH, OA, O[C(R³)₂]ₙAr, O[C(R³)₂]ₙHet, N(R³)₂, NR³[C(R³)₂]ₙAr oder NR³[C(R³)₂]ₙHet,
- R²: einen ungesättigten oder aromatischen fünf- oder sechsgliedrigen Heterocyclus mit 1 bis 4 N-, O- und/oder S-Atomen, der unsubstituiert oder ein- oder zweifach durch Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R')₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA und/oder =O (Carbonylsauerstoff) substituiert sein kann,
- R³: H oder A',
- R⁴: H, A oder Hal,
- A: unverzweigtes oder verzweigtes Alkyl mit 1-10 C-Atomen, worin 1-7 H-Atome durch OH, F, Cl und/oder Br ersetzt sein können, und/oder worin eine oder zwei CH₂-Gruppen durch O, NH, S, SO, SO₂ und/oder CH=CH-Gruppen ersetzt sein können, oder cyclisches Alkyl mit 3-7 C-Atomen, worin 1-7 H-Atome durch OH, F, Cl und/oder Br ersetzt sein können,
- A': unverzweigtes oder verzweigtes Alkyl mit 1-6 C-Atomen, worin 1-5 H-Atome durch F ersetzt sein können,
- Ar: unsubstituiertes oder ein-, zwei- oder dreifach durch Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂)ₙHet¹, OCONH[C(R³)₂]ₚN(R³)₂ und/oder OCONH[C(R³)₂]ₙHet¹ substituiertes Phenyl, Naphthyl oder Biphenyl,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, N(R³)₂, SR³, NO₂, CN, COOR³, CON(R³)₂, NR³COA, NR³SO₂A, SO₂N(R³)₂, S(O)ₘA, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA und/oder =O (Carbonylsauerstoff) (Carbonylsauerstoff) substituiert sein kann,
- Het ¹: einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
- Hal: F, Cl, Br oder I,
- m: 0, 1 oder 2,
- n: 0, 1, 2, 3 oder 4,
- p: 2, 3, 4, 5, oder 6,
bedeuten,
sowie ihre pharmazeutisch verwendbaren Salze, Tautomere und Stereoisomere, einschließlich deren Mischungen in allen Verhältnissen, Gegenstand der Erfindung sind auch die optisch aktiven Formen (Stereo-isomeren), die Enantiomeren, die Racemate sowie die Diastereomeren dieser Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen. Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-12 sowie ihrer pharmazeutisch verwendbaren Salze, Tautomeren und Stereoisomeren, dadurch gekennzeichnet, daß man
eine Verbindung der Formel II 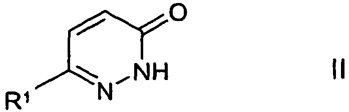
worin R¹ die in Anspruch 1 angegebene Bedeutung hat,
mit einer Verbindung der Formel III 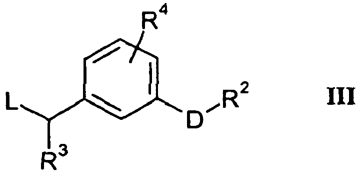
worin D, R², R³ und R⁴ die in Anspruch 1 angegebenen Bedeutungen haben und
L Cl, Br, I oder eine freie oder reaktionsfähig funktionell abgewandelte OH-Gruppe bedeutet,
umsetzt,

und/oder eine Base oder Säure der Formel I in eines ihrer Salze umwandelt.

Vor- und nachstehend haben die Reste D, R¹, R², R³ und R⁴ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. Cyclisches Alkyl (Cycloalkyl) bedeutet vorzugsweise Cyclopropyl, Cyclobutyl, Cylopentyl, Cyclohexyl oder Cycloheptyl.
A bedeutet weiterhin unverzweigtes oder verzweigtes Alkyl mit 1-6 C-Atomen, worin auch eine oder zwei CH₂-Gruppen durch O ersetzt sein können. A bedeutet daher vorzugsweise auch 2-Hydroxyethyl, 3-Hydroxypropyl, 2-Methoxyethyl oder 3-Methoxypropyl.

A' bedeutet Alkyl, dieses ist unverzweigt (linear) oder verzweigt, und hat 1, 2, 3, 4, 5 oder 6 C-Atome. A' bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A' bedeutet ganz besonders bevorzugt Methyl, Ethyl, Propyl, Isopropyl, Butyl, Isobutyl, sek.-Butyl, tert.-Butyl, Pentyl, Hexyl oder Trifluormethyl.

R¹ bedeutet vorzugsweise Ar oder Het.
R² bedeutet vorzugsweise einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl.
R³ bedeutet vorzugsweise H oder Methyl.
R⁴ bedeutet vorzugsweise H.

n bedeutet vorzugsweise 0, 1 oder 2.
p bedeutet vorzugsweise 2 oder 3.
q bedeutet vorzugsweise 1, 2, 3 oder 4, ganz besonders bevorzugt 1.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Aminosulfonylphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet besonders bevorzugt ein-, zwei-oder dreifach durch Hal und/oder CN substituiertes Phenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-. 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzo-dioxol-5-yl, 1,4-Benzodioxan-6-yl, 2,1,3-Benzothiadiazol-4-oder-5-yl, 2,1,3-Benzoxadiazol-5-yl oder Dibenzofuranyl.
Die heterocyclischen Reste können auch teilweise oder vollständig hydriert sein.
Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, - 6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)-phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1 H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet besonders bevorzugt einen einkernigen aromatischen Heterocyclus mit 1 bis 4 N-, O- und/oder S-Atomen, der unsubstituiert oder ein- oder zweifach durch A substituiert sein kann.

Het bedeutet ganz besonders bevorzugt Pyrazolyl, Pyridinyl, Pyrimidinyl, Furyl, Thienyl, Oxazolyl, Oxadiazolyl, Imidazolyl, Pyrrolyl oder Isoxazolyl, wobei die Reste auch ein- oder zweifach durch A substituiert sein können.

Het¹ bedeutet vorzugsweise Piperidinyl, Pyrrolidinyl, Morpholinyl, Piperazinyl, Oxazolidinyl oder Imidazolidinyl, wobei die Reste auch ein- oder zweifach durch =O und/oder A substituiert sein können.
Het¹ bedeutet ganz besonders bevorzugt Piperidinyl, Pyrrolidinyl, Morpholinyl, Piperazinyl, Oxazolidinyl oder Imidazolidinyl, wobei die Reste auch einfach durch A substituiert sein können.

D bedeutet vorzugsweise Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl, wobei die Reste auch ein-, zwei- oder dreifach durch Hal und/oder A substituiert sein können, ganz besonders bevorzugt Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis II ausgedrückt werden, die der Formel I entsprechen und worin die nicht näher bezeichneten Reste die bei der Formel I angegebene Bedeutung haben, worin jedoch
in la
   - D: Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
   bedeutet;
in Ib
   - R¹: Ar oder Het bedeutet;
in Ic
   - R²: einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl
   bedeutet;
in Id
   - R³: H oder Methyl bedeutet;
in le
   - R⁴: H bedeutet;
in If
   - Ar: ein-, zwei-oder dreifach durch Hal und/oder CN substituiertes Phenyl
   bedeutet;
in Ig
   - A: unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
   bedeutet;
in Ih
   - Het: einen einkernigen aromatischen Heterocyclus mit 1 bis 4 N-, O- und/oder S-Atomen, der unsubstituiert oder ein- oder zweifach durch A substituiert sein kann,
   bedeutet;
in li
   - Het: Pyrazolyl, Pyridinyl, Pyrimidinyl, Furyl, Thienyl, Oxazolyl, Oxadiazolyl, Imidazolyl, Pyrrolyl oder Isoxazolyl, wobei die Reste auch ein- oder zweifach durch A substituiert sein können,
   bedeutet;
in Ij
   - Het¹: Piperidinyl, Pyrrolidinyl, Morpholinyl, Piperazinyl, Oxazolidinyl oder Imidazolidinyl, wobei die Reste auch ein- oder zweifach durch =O und/oder A substituiert sein können,
   bedeuten;
in lk
   - D: Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
   - R¹: Ar oder Het,
   - R²: einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl,
   - R³: H oder Methyl,
   - R⁴: H,
   - A: unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
   - Ar: ein-, zwei-oder dreifach durch Hal und/oder CN substituiertes Phenyl,
   - Het: einen einkernigen aromatischen Heterocyclus mit 1 bis 4 N-, O- und/oder S-Atomen, der unsubstituiert oder ein- oder zweifach durch A substituiert sein kann,
   - Het¹: einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
   - Hal: F, Cl, Br oder I,
   - n: 0, 1, 2, 3 oder 4
   bedeuten;
in II
   - D: Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
   - R¹: Ar oder Het,
   - R²: einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl,
   - R³: H oder Methyl,
   - R⁴: H,
   - A: unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
   - Ar: ein-, zwei-oder dreifach durch Hal und/oder CN substituiertes Phenyl,
   - Het: Pyrazolyl, Pyridinyl, Pyrimidinyl, Furyl, Thienyl, Oxazolyl, Oxadiazolyl, Imidazolyl, Pyrrolyl oder Isoxazolyl, wobei die Reste auch ein- oder zweifach durch A substituiert sein können,
   - Het¹: Piperidinyl, Pyrrolidinyl, Morpholinyl, Piperazinyl, Oxazolidinyl oder Imidazolidinyl, wobei die Reste auch ein- oder zweifach durch =O und/oder A substituiert sein können,
   - Hal: F, Cl, Br oder I,
   - n: 0, 1, 2, 3 oder 4,
   bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man eine Verbindung der Formel II mit einer Verbindung der Formel III umsetzt. In den Verbindungen der Formel III bedeutet L vorzugsweise OH, Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Ethanol, Toluol, Dimethoxyethan oder THF.

Besonders bevorzugt ist die Umsetzung mit Triphenylphosphin und einem Azodicarboxylat, falls in den Verbindungen der Formel III L OH bedeutet. In diesem Fall erfolgt die Umsetzung vorzugsweise in THF bei Temperaturen zwischen -15 und 5 °.

Verbindungen der Formel I können weiterhin vorzugsweise erhalten werden, indem man einen Rest R¹ und/oder R² in einen anderen Rest R¹ und/oder R² umwandelt,
indem man
i) eine Halogen- oder Hydroxygruppe durch einen Alkyl-, einen heterocyclischen Rest oder einen Arylrest ersetzt,
ii) eine Carboxygruppe zu einem Amid umsetzt,
iii) ein Amin alkyliert,
iv) eine Hydroxygruppe verethert.

Der Austausch eines Halogenatoms erfolgt vorzugsweise unter den Bedingungen einer Suzuki-Kopplung.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°. Die Tritylgruppe wird zum Schutz der Aminosäuren Histidin, Asparagin, Glutamin und Cystein eingesetzt. Die Abspaltung erfolgt, je nach gewünschtem Endprodukt, mit TFA /10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt. Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanot/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie deren Salze davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per* se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst in dieser Offenbarung ist die Verwendung der erfindungsgemäßen Verbindungen nach Anspruch 1 und/oder ihre physiologisch unbedenklichen Salze zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Offenbarung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst in dieser Offenbarung ist die Verwendung der Verbindungen der Formel I und/oder ihre physiologisch unbedenklichen Salze zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Offenbarung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Offenbarung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Offenbarung.

Der Ausdruck "tyrosinkinasebedingte Krankheiten oder Leiden" bezieht sich auf pathologische Zustände, die von der Aktivität einer oder mehrerer Tyrosin-kinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs, insbesondere schnell wachsenden Tumoren, verabreicht werden.

Gegenstand der Offenbarung ist somit die Verwendung von Verbindungen der Formel I, sowie ihrer pharmazeutisch verwendbaren Salze und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Met-Kinase.

Bevorzugt ist die Verwendung von Verbindungen der Formel I, sowie ihrer pharmazeutisch verwendbaren Salze und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden.
Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthe |
| | Tetraplatin | BBR-3464 (Hoffrnann-La Roche) |
| | Ormiplatin | |
| | Iproplatin | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche |
| | Idatrexate | Ethinylcytidin (Taiho) |
| | | |
| Topoisomerase- | Amsacrin | Rubitecan (SuperGen) |
| Inhibitoren | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10- | |
| | hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| | | |
| Antitumor-Antibiotik | Dactinomycin (Actinomycin | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubici | GPX-100 (Gem Pharmaceuticals) |
| | Mitoxantron (Novantron) | |
| | | |
| Antimitotische Mittel | Paclitaxel | SB 408075 (GlaxoSmithKlin |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient |
| | Vinflunin (Fabre) | NeuroPharma) |
| | Auristatin PE (Teikoku Hormone) | Azaepothilon B (BMS) |
| | | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase- | Aminoglutethimid | Exemestan |
| Inhibitoren | Letrozol | Atamestan (BioMedicines) |
| | Anastrazol | YM-511 (Yamanouchi) |
| | Formestan | |
| | | |
| Thymidylatsynthase | Pemetrexed (Eli Lilly) | Nolatrexed (Eximias) |
| Inhibitoren | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum |
| | Albumin + 32P (Isotope Solutions) | Pharmaceuticals) |
| | | O6-Benzylguanin (Paligent) |
| | Thymectacin (NewBiotics) | |
| | Edotreotid (Novartis) | |
| | | |
| Farnesyltransferase | Arglabin (NuOncology Labs) | Tipifarnib (Johnson & Johnson) |
| Inhibitoren | Ionafarnib (Schering-Plough BAY-43-9006 (Bayer) | |
| | | Perillylalkohol (DOR BioPharma) |
| | | |
| Pumpen-Inhibitorer | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltrans- | Tacedinalin (Pfizer) | Pivaloyloxymethylbutyrat (Titan) |
| ferase-Inhibitoren | SAHA (Aton Pharma) | |
| | MS-275 (Schering AG) | Depsipeptid (Fujisawa) |
| | | |
| Metalloproteinase- | Neovastat (Aeterna | CMT -3 (CollaGenex) |
| Inhibitoren | Laboratories) | BMS-275291 (Celltech) |
| Ribonucleosidredul | Marimastat (British Biotech) | Tezacitabin (Aventis) |
| se- | Galliummaltolat (Titan) | Didox (Molecules for Health |
| Inhibitoren | Triapin (Vion) | |
| | | |
| TNF-alpha-Agonisten / Anta- | Virulizin (Lorus Therapeutics | Revimid (Celgene) |
| | CDC-394 (Celgene) | |
| gonisten | | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatorer | Interferon | Dexosom-Therapie (Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa: | |
| | | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | |
| | Dexamethason | Arzoxifen (Eli Lilly) |
| | | |
| Photodynamische | Talaporfin (Light Sciences) | Pd-Bacteriopheophorbid (Yeda) |
| Mittel | Theralux (Theratechnologie: | |
| | Motexafin-Gadolinium (Pharmacyclics) | Lutetium-Texaphyrin (Pharmacyclics) |
| | | Hypericin |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| Verschiedene Mitte | SR-27897 (CCK-A-Inhibitor, Sanofi-Synthelabo) | BCX-1777 (PNP-Inhibitor, BioCryst) |
| | Tocladesin (cyclisches-AMP Agonist, Ribapharm) | Ranpirnase (Ribonuclease-Stimulans, Alfacell) |
| | Alvocidib (CDK-Inhibitor, Aventis) | Galarubicin (RNA-Synthese Inhibitor, Dong-A) |
| | CV-247 (COX-2-Inhibitor, Iv Medical) | Tirapazamin |
| | | (Reduktionsmittel, SRI |
| | P54 (COX-2-Inhibitor, Phytopharm) | International) |
| | | N-Acetylcystein |
| | CapCell™ (CYP450- | (Reduktionsmittel, Zambon) |
| | Stimulans, Bavarian Nordic) | R-Flurbiprofen (NF-kappaB- |
| | GCS-100 (gal3-Antagonist, GlycoGenesys) | Inhibitor, Encore) |
| | | 3CPA (NF-kappaB-Inhibitor |
| | G17DT-Immunogen (Gastrin Inhibitor, Aphton) | Active Biotech) |
| | | Seocalcitol (Vitamin-D- |
| | Efaproxiral (Oxygenator, All Therapeutics) | Rezeptor-Agonist, Leo) |
| | | 131-1-TM-601 (DNA- |
| | PI-88 (Heparanase-Inhibitor Progen) | Antagonist, TransMolecular) Eflornithin (ODC-Inhibitor, ILEX Oncology) |
| | Tesmilifen (Histamin-Antagonist, YM BioScience: | |
| | | Minodronsäure (Osteoclaste Inhibitor, Yamanouchi) |
| | Histamin (Histamin-H2-Rezeptor- Agonist, Maxim) | |
| | | Indisulam (p53-Stimulans, Eisai) |
| | Tiazofurin (IMPDH-Inhibitor, Ribapharm) | |
| | | Aplidin (PPT-Inhibitor, PharmaMar) |
| | Cilengitid (Integrin-Antagoni Merck KGaA) | |
| | | Rituximab (CD20-Antikörper Genentech) |
| | SR-31747 (IL-1-Antagonist. Sanofi-Synthelabo) | |
| | | Gemtuzumab (CD33-Antikörper, Wyeth Ayerst) |
| | CCI-779 (mTOR-Kinase- | |
| | Inhibitor, Wyeth) | PG2 (Hämatopoese- |
| | Exisulind (PDE-V-Inhibitor, Cell Pathways) | Verstärker, Pharmagenesis) |
| | | Immunol™ (Triclosan-Oralspülung, Endo) |
| | CP-461 (PDE-V-Inhibitor, Co Pathways) | |
| | | Triacetyluridin (Uridin-Prodr Wellstat) |
| | AG-2037 (GART-Inhibitor, Pfizer) | |
| | | SN-4071 (Sarkom-Mittel, |
| | WX-UK1 | Signature BioScience) |
| | (Plasminogenaktivator-Inhibitor, Wilex) | TransMID-107™ (Immunotoxin, KS Biomedix |
| | PBI-1402 (PMN-Stimulans, ProMetic LifeSciences) | PCK-3145 (Apoptose-Förderer, Procyon) |
| | Bortezomib (Proteasom-Inhibitor, Millennium) | Doranidazol (Apoptose-Förderer, Pola) |
| | SRL-172 (T-Zell-Stimulans, SR Pharma) | CHS-828 (cytotoxisches Mittel, Leo) |
| | TLK-286 (Glutathion-S-Transferase-Inhibitor, Telik) | trans-Retinsäure (Differentiator, NIH) |
| | PT-100 (Wachstumsfaktor-Agonist, Point Therapeutics) | MX6 (Apoptose-Förderer, MAXIA) |
| | Midostaurin (PKC-Inhibitor, Novartis) | Apomin (Apoptose-Förderer ILEX Oncology) |
| | Bryostatin-1 (PKC-Stimulan: GPC Biotech) | Urocidin (Apoptose-Fördere Bioniche) |
| | CDA-II (Apoptose-Förderer, Everlife) | Ro-31-7453 (Apoptose-Förderer, La Roche) |
| | SDX-101 (Apoptose-Fördere Salmedix) | Brostallicin (Apoptose-Förderer, Pharmacia) |
| | | |
| | Ceflatonin (Apoptose-Förderer, ChemGenex) | |
| | | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthe |
| | Tetraplatin | BBR-3464 (Hoffrnann-La Roche) |
| | Ormiplatin | |
| | Iproplatin | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharma) |
| | Methotrexat | DMDC (Hoffmann-La Roche |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Ditlomotecan (Beaufour-Ipsen) |
| | 7-Ethyl-10- | |
| | hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharma) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| | | |
| Antitumor-Antibiotif | Dactinomycin (Actinomycin I | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubici | GPX-100 (Gem |
| | Mitoxantron (Novantron) | Pharmaceuticals) |
| Antimitotische Mittel | Paclitaxel | SB 408075 (GlaxoSmithKlin |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCl) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharma) |
| | Vinflunin (Fabre) | |
| | Auristatin PE (Teikoku Hormone) | Azaepothilon B (BMS) |
| | | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum |
| | Albumin + 32P (Isotope Solutions) | Pharmaceuticals) |
| | | O6-Benzylguanin (Paligent) |
| | Thymectacin (NewBiotics) | |
| | Edotreotid (Novartis) | |
| | | |
| Farnesyltransferase Inhibitoren | Arglabin (NuOncology Labs) | Tipifarnib (Johnson & Johnson) |
| | Ionafarnib (Schering-Plough | |
| | BAY-43-9006 (Bayer) | Perillylalkohol (DOR BioPharma) |
| Pumpen-Inhibitorer | CBT-1 (CBA Pharma) | Zosuquidar-Trihydrochlorid (Eli Lilly) |
| | Tariquidar (Xenova) | |
| | MS-209 (Scherinq AG) | Biricodar-Dicitrat (Vertex) |
| | | |
| Histonacetyltransferase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidredul se-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten/Antagon en | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatorer | Interferon | Dexosom-Therapie (Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa: | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologie | |
| | Motexafin-Gadolinium (Pharmacyclics) | Lutetium-Texaphyrin (Pharmacyclics) |
| | | |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1 C11 (ImClone) |
| | EKB-509 (Wyeth) | |
| | EKB-569 (Wyeth) | |
| | | |
| Verschiedene Mitte | SR-27897 (CCK-A-Inhibitor Sanofi-Synthelabo) | BCX-1777 (PNP-Inhibitor, BioCryst) |
| | Tocladesin (cyclisches-AMF Agonist, Ribapharm) | Ranpirnase (Ribonuclease-Stimulans, Alfacell) |
| | Alvocidib (CDK-Inhibitor, Aventis) | Galarubicin (RNA-Synthese-Inhibitor, Dong-A) |
| | CV-247 (COX-2-Inhibitor, Iv | Tirapazamin (Reduktionsmit |
| | Medical) | SRI International) |
| | P54 (COX-2-Inhibitor, Phytopharm) | N-Acetylcystein (Reduktionsmittel, Zambon) |
| | CapCell™ (CYP450-Stimulans, Bavarian Nordic GCS-IOO (gal3-Antagonist, GlycoGenesys) | R-Flurbiprofen (NF-kappaB-Inhibitor, Encore) |
| | | 3CPA (NF-kappaB-Inhibitor, Active Biotech) |
| | G17DT-Immunogen (Gastri Inhibitor, Aphton) | Seocalcitol (Vitamin-D-Rezeptor-Agonist, Leo) |
| | Efaproxiral (Oxygenator, All Therapeutics) | 131-I-TM-601 (DNA-Antagonist, TransMolecular) |
| | PI-88 (Heparanase-Inhibito Progen) | Eflornithin (ODC-Inhibitor, IL Oncology) |
| | Tesmilifen (Histamin-Antagonist, YM BioScience | Minodronsäure (Osteoclaste Inhibitor, Yamanouchi) |
| | Histamin (Histamin-H2-Rezeptor- Agonist, Maxim) | Indisulam (p53-Stimulans, Eisai) |
| | Tiazofurin (IMPDH-Inhibitor Ribapharm) | Aplidin (PPT-Inhibitor, PharmaMar) |
| | Cilengitid (Integrin-Antagonist, Merck KGaA) | Rituximab (CD20-Antikörper Genentech) |
| | SR-31747 (IL-1-Antagonist, Sanofi-Synthelabo) | Gemtuzumab (CD33-Antikörper, Wyeth Ayerst) |
| | CCI-779 (mTOR-Kinase-Inhibitor, Wyeth) | PG2 (Hämatopoese-Verstärker, Pharmagenesis) |
| | Exisulind (PDE-V-Inhibitor, Cell Pathways) | Immunol™ (Triclosan-Oralspülung, Endo) |
| | CP-461 (PDE-V-Inhibitor, C Pathways) | Triacetyluridin (Uridin-Prodru Wellstat) |
| | AG-2037 (GART-Inhibitor, Pfizer) | SN-4071 (Sarkom-Mittel, Signature BioScience) |
| | WX-UK1 (Plasminogenaktivator-Inhibitor, Wilex) | TransMID-107™ (Immunotoxin, KS Biomedix) PCK-3145 (Apoptose-Förder Procyon) |
| | PBI-1402 (PMN-Stimulans, ProMetic LifeSciences) | |
| | | Doranidazol (Apoptose- |
| | Bortezomib (Proteasom-Inhibitor, Millennium) | Förderer, Pola) |
| | | CHS-828 (cytotoxisches Mitt Leo) |
| | SRL-172 (T-Zell-Stimulans, SR Pharma) | |
| | | trans-Retinsäure (Differentiator, NIH) |
| | TLK-286 (Glutathion-S-Transferase-Inhibitor, Telik) | |
| | | MX6 (Apoptose-Förderer, MAXIA) |
| | PT-100 (Wachstumsfaktor-Agonist, Point Therapeutics | |
| | | Apomin (Apoptose-Förderer !LEX Oncology) |
| | Midostaurin (PKC-Inhibitor, Novartis) | |
| | | Urocidin (Apoptose-Förderer |
| | Bryostatin-1 (PKC-Stimulan | Bioniche) |
| | GPC Biotech) | Ro-31-7453 (Apoptose-Förderer, La Roche) |
| | CDA-II (Apoptose-Förderer Everlife) | |
| | | Brostallicin (Apoptose-Förderer, Pharmacia) |
| | SDX-101 (Apoptose-Förderer, Salmedix) | |
| | | |
| | Ceflatonin (Apoptose-Förderer, ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Girnbrone et al., J. Natl. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity-(Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214). Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidasekonjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.
Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer). Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer;
   (10 ng Enzym/well, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

### In vivo-Tests

Experimenteller Ablauf: Weibliche Balb/C Mäuse (Züchter: Charles River Wiga) waren bei der Ankunft im Alter von 5 Wochen. Sie wurden 7 Tage lang an unsere Haltungsbedingungen akklimatisiert. Anschließend wurden jeder Maus 4 Millionen TPR-Met / NIH3T3 - Zellen in 100 µl PBS (ohne Ca++ und Mg++) subkutan im Beckenbereich injiziert. Nach 5 Tagen wurden die Tiere in 3 Gruppen randomisiert, so dass jede Gruppe von 9 Mäusen ein mittleres Tumorvolumen von 110 µl (Spanne: 55 - 165) hatte. Der Kontrollgruppe wurden 100 µl Vehikel (0,25 % Methylzellulose / 100 mM Acetatpuffer, pH 5.5), den Behandlungsgruppen wurden 200 mg/kg "A56" bzw. "A91" gelöst im Vehikel (Volumen ebenfalls 100 µl / Tier) per Schlundsonde täglich verabreicht. Nach 9 Tagen hatten die Kontrollen ein mittleres Volumen von 1530 µl und der Versuch wurde beendet.

Messung des Tumorvolumens: Die Länge (L) und Breite (B) wurde mit einer Schubleere gemessen und das Tumorvolumen nach der Formel LxBxB/2 berechnet.

Haltungsbedingungen: je 4 bzw. 5 Tiere pro Käfig, Fütterung mit kommerziellem Mäusefutter (Fa. Sniff).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1;

Schmelzpunkt F. in °C.
- Massenspektrometrie (MS):: El (Elektronenstoß-Ionisation) M⁺ FAB (Fast Atom Bombardment) (M+H)⁺
ESI (Electrospray lonization) (M+H)⁺
APCI-MS (atmospheric pressure chemical ionization - mass spectrometry) (M+H)⁺.

### HPLC-Methoden:

**Methode A:** Gradient: 4,5 min/ FI.: 3 ml/min 99:01 - 0:100
   Wasser+0.1 %(Vol.)TFA : Acetonitril+0.1 %(Vol.)TFA
   0.0 bis 0.5 min: 99:01
   0.5 bis 3.5 min: 99:01 -> 0:100
   3.5 bis 4.5 min: 0:100
   Säule: Chromolith SpeedROD RP18e 50-4.6
   Wellenlänge: 220nm
**Methode B:** Gradient: 4.2 min/ Fluss: 2 ml/min 99:01 - 0:100
   Wasser + 0.1%(Vol.) TFA : Acetonitril + 0.1%(Vol.) TFA
   0.0 bis 0.2 min: 99:01
   0.2 bis 3.8 min: 99:01 ---> 0:100
   3.8 bis 4.2 min: 0:100
   Säule: Chromolith Performance RP18e; 100 mm lang,
   Innendurchmesser 3 mm
   Wellenlänge: 220nm
Retentionszeit Rt. in Minuten [min].

### Beispiel 1

Die Herstellung von 6-(3,5-Difluor-phenyl)-2-{3-[5-(1-piperidin-4-yl-1H-pyrazol-4-yl)-thiazol-2-yi]-benzyll-2H-pyridazin-3-on ("A1") erfolgt analog nachstehendem Schema 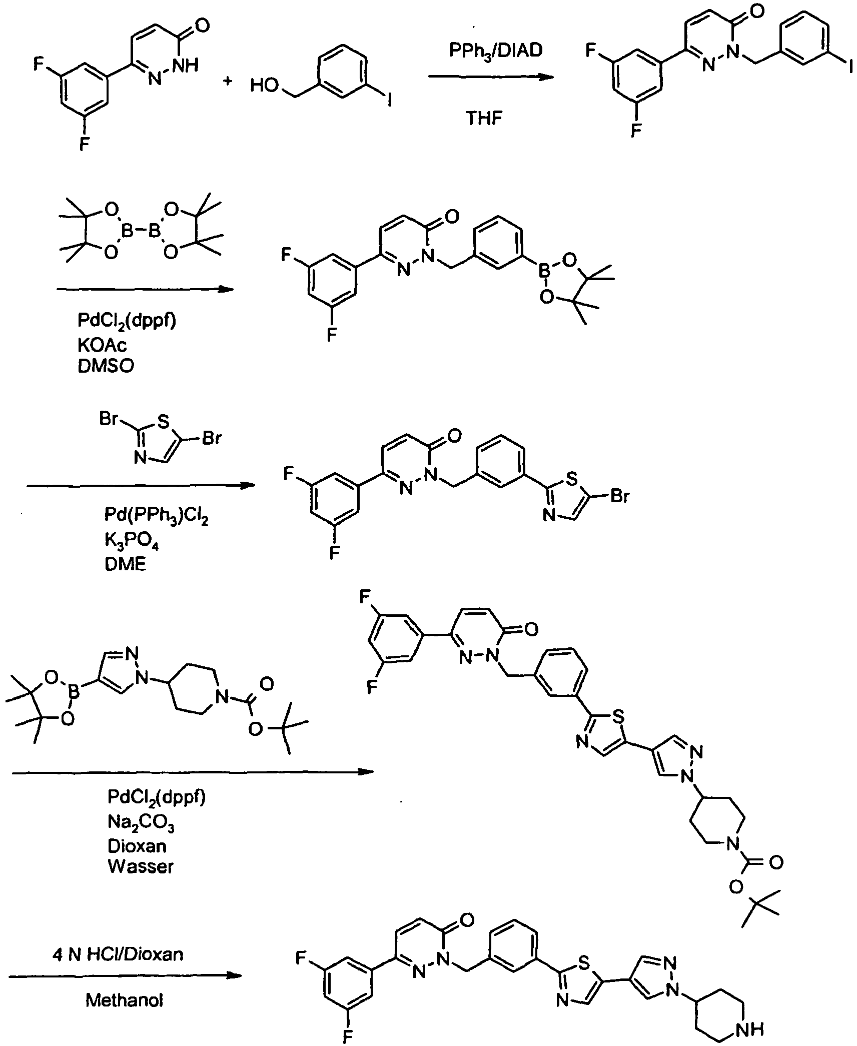
1.1 Eine Suspension von 5.81 g (27.9 mmol) 6-(3,5-Difluorphenyl)-2H-pyridazin-3-on (hergestellt nach WO2008/017361) in 120 ml THF wird mit 10.0 g (41.9 mmol) 3-Iodbenzylalkohol und 11.1 g (41.9 mmol) Triphenylphosphin versetzt und im Eisbad auf 0° C gekühlt. 8.65 ml (41.9 mmol) Diisopropylazodicarboxylat werden unter Rühren langsam zugetropft und das Reaktionsgemisch 3 Stunden bei Raumtemperatur gerührt. Das Reaktionsgemisch wird eingedampft, der Rückstand wird in heißem 2-Propanol gelöst und auf Raumtemperatur gekühlt. Der entstandene Feststoff wird abgesaugt, mit 2-Propanol gewaschen und getrocknet. Das Rohprodukt wird noch mal aus 2-Propanol umkristallisiert: 6-(3,5-Difluorphenyl)-2-(3-iodbenzyl)-2H-pyridazin-3-on als gelbliche Kristalle; ESI 425.
1.2 Eine unter Stickstoff gehaltene Lösung von 8.48 g (20.0 mmol) 6-(3,5-Difluorphenyl)-2-(3-iodbenzyl)-2H-pyridazin-3-on und 6.10 g (24.0 mmol) Bis(pinacolato)-dibor in 40 ml Dimethylsulfoxid wird mit 6.67 g (68.0 mmol) Kaliumacetat und 817 mg (1.00 mmol) 1,1-Bis(diphenylphosphino)-ferrocen-dichloropalladium(II) versetzt, auf 80° C erhitzt und 6 Stunden bei dieser Temperatur gerührt. Das Reaktionsgemisch wird auf Raumtemperatur gekühlt, mit Wasser versetzt und der entstandene Niederschlag durch Abdekantieren der überstehenden Lösung isoliert. Dieser Rückstand wird zwischen Wasser und Dichlormethan verteilt. Die organische Phase wird über Natriumsulfat getrocknet, eingedampft und der Rückstand an einer Kieselgelsäule mit Dichlormethan/Methanol chromatographiert: 6-(3,5-Difluorphenyl)-2-[3-(4,4,5,5-tetramethyl-[1,3,2]dioxaborolan-2-yl)-benzyl]-2H-pyridazin-3-on als leicht graue Kristalle; ESI 425.
1.3 Eine unter Stickstoff gehaltene Lösung von 848 mg (2.00 mmol) 6-(3,5-Difluorphenyl)-2-[3-(4,4,5,5-tetramethyl-[1,3,2]dioxaborolan-2-yl)-benzyl]-2H-pyridazin-3-on und 455 mg (1.82 mmol) 2,5-Dibromthiazol in 18 ml 1,2-Dimethoxyethan wird mit 772 mg (3.64 mmol) Trikaliumtriphosphat-Trihydrat und 102 mg (0.15 mmol) Bis(triphenylphosphin)-palladium(II)-chlorid versetzt, auf 80° C erhitzt und 42 Stunden bei dieser Temperatur gerührt. Das Reaktionsgemisch wird auf Raumtemperatur gekühlt, filtriert und das Filtrat zwischen Wasser und Ethylacetat verteilt. Die organische Phase wird über Natriumsulfat getrocknet, eingedampft und der Rückstand an einer Kieselgelsäule mit Petrolether/Ethylacetat chromatographiert: 2-[3-(5-Bromthiazol-2-yl)-benzyl]-6-(3,5-difluorphenyl)-2H-pyridazin-3-on als gelber Feststoff; ESI 460,462.
1.4 Eine unter Stickstoff gehaltene Lösung von 230 mg (0.50 mmol) 2-[3-(5-Bromthiazol-2-yl)-benzyl]-6-(3,5-difluorphenyl)-2H-pyridazin-3-on und208 mg (0.55 mmol) 4-[4-(4,4,5,5-Tetramethyl-[1,3,2]dioxaborolan-2-yl)-pyrazol-1-yl]-piperidin-1-carbonsäure-tert-butylester (hergestellt nach WO2007/066187) in 5 ml Dioxan wird mit 106 mg (1.00 mmol) Natriumcarbonat, 0.5 ml Wasser und 18.3 mg (0.025 mmol) 1,1-Bis(diphenyl-phosphino)ferrocen-dichloropalladium(II) versetzt und 42 Stunden bei 80° C gerührt. Das Reaktionsgemisch wird auf Raumtemperatur gekühlt, filtriert und der Rückstand an einer Kieselgelsäule mit Dichlormethan/Methanol als Laufmittel chromatographiert: 4-[4-(2-{3-[3-(3,5-Difluorphenyl)-6-oxo-6H-pyridazin-1-ylmethyl]-phenyl}-thiazol-5-yl)-pyrazol-1-yl]-piperidin-1-carbonsäure-tert-butylester als gelbe Kristalle; ESI 631.
1.5 Eine Suspension von 156 mg (0.25 mmol) 4-[4-(2-{3-[3-(3,5-Difluorphenyl)-6-oxo-6H-pyridazin-1-ylmethyl]-phenyl}-thiazol-5-yl)-pyrazol-1 -yl]-piperidin-1-carbonsäure-tert.-butylester in einem Gemisch von 4 ml 10%iger HCl in Dioxan und 1 ml Methanol wird kurz zum Sieden erhitzt und sechs Stunden bei Raumtemperatur gerührt. Das Reaktionsgemisch wird eingedampft und der Rückstand zwischen 2 N Natronlauge und Dichlormethan verteilt. Die organische Phase wird über Natriumsulfat getrocknet, eingedampft und der Rückstand mit tert.-Butylmethylether verrührt: 6-(3,5-Difluorphenyl)-2-{3-[5-(1-piperidin-4-yl-1H-pyrazol-4-yl)-thiazol-2-yl]-benzyl}-2H-pyridazin-3-on als gelber Feststoff; ESI 531;
   ¹H-NMR (d₆-DMSO) δ [ppm] 1.87 (m, 2H), 2.03 (m, 2H), 2.70 (m, 2H), 3.13 (m, 2H), 4.28 (m, 1H), 5.43 (s, 2H), 7.15 (d, J = 10 Hz, 1H), 7.35 (tt, J₁ = 8.8 Hz, J₂ = 2.3 Hz, 1 H), 7.49 (m, 2H), 7.67 (m, 2H), 7.83 (m, 2H), 7.99 (s, 1H), 8.01 (s, 1H), 8.15 (d, J = 10 Hz, 1H), 8.23 (s, 1H).

### Beispiel 2

Die Herstellung von 6-(3-Fluor-phenyl)-2-{3-[5-(1 -piperidin-4-yl-1H-pyrazol-4-yl)-thiazol-2-yl]-benzyl}-2H-pyridazin-3-on ("A2") erfolgt analog nachstehendem Schema 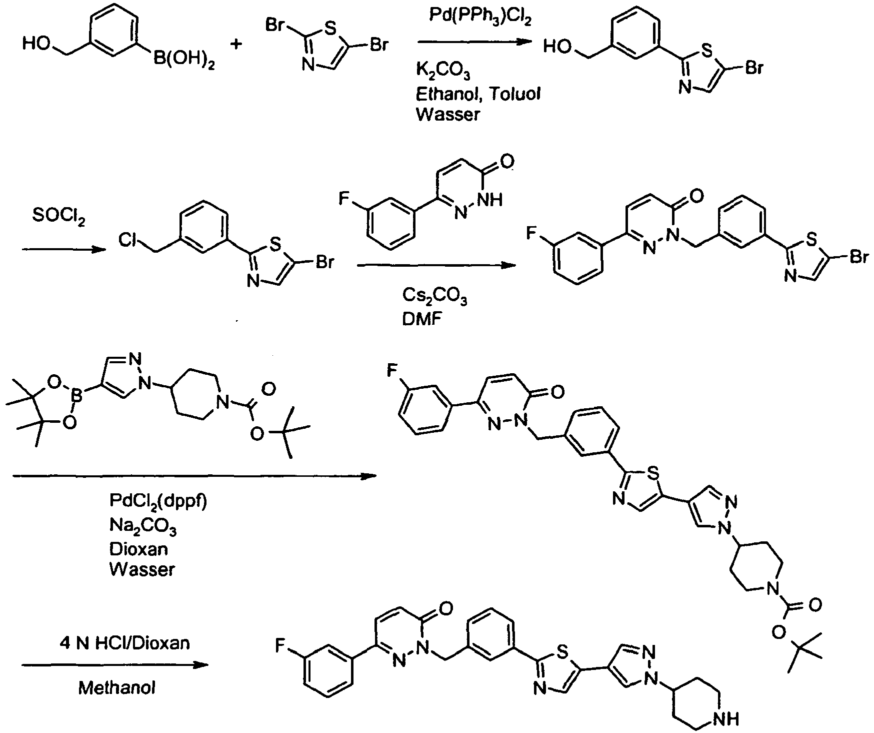
2.1 Eine unter Stickstoff gehaltene Lösung von 6.79 g (28.0 mmol) 2,5-Dibromthiazol in 28 ml Toluol wird mit einer Lösung von 5.80 g (41.9 mmol) Kaliumcarbonat in 28 ml Wasser versetzt und das Gemisch auf 80° C erhitzt. Dazu werden 196 mg (0.28 mmol) Bis(triphenylphosphin)palladium(II)-chlorid gegeben und anschließend eine Lösung von 4.67 g (30.7 mmol) 3-(Hydroxymethyl)-benzolboronsäure in 56 ml Ethanol zugetropft. Das Reaktionsgemisch wird 18 Stunden bei 80° C gerührt. Das Reaktionsgemisch wird im Vakuum eingeengt und der Rückstand zwischen Ethylacetat und Wasser verteilt. Die organische Phase wird über Natriumsulfat getrocknet, eingedampft und der Rückstand an einer Kieselgelsäule mit Dichlormethan/Methanol als Laufmittel chromatographiert: 3-(5-Brom-thiazol-2-yl)-phenyl]-methanol als braunes Öl; ESI 270,272.
2.2 Zu 1 ml Thionylchlorid wird unter Rühren und externer Eisbadkühlung portionsweise 454 mg (1.68 mmol) 3-(5-Bromthiazol-2-yl)-phenyl]-methanol gegeben und das Reaktionsgemisch 18 Stunden bei Raumtemperatur gerührt. Das Reaktionsgemisch wird mit tert.-Butylmethylether und Toluol verdünnt und eingedampft. Der Rückstand wird mit tert.-Butylmethylether verrührt: 5-Brom-2-(3-chlormethyl-phenyl)-thiazol als grauer Feststoff; ESI 290.
2.3 Eine Lösung von 85.1 mg (0.30 mmol) 5-Brom-2-(3-chlormethylphenyl)-thiazol und 61.7 mg (0.32 mmol) 6-(3-Fluorphenyl)-2H-pyridazin-3-on in 0.5 ml DMF wird mit 96 mg (0.30 mmol) Caesiumcarbonat versetzt und die entstandene Suspension 18 Stunden bei Raumtemperatur gerührt. Das Reaktionsgemisch wird mit Wasser und Dichlormethan versetzt. Die organische Phase wird abgetrennt, über Natriumsulfat getrocknet und eingedampft. Der Rückstand wird aus heißem tert.-Butylmethylether kristallisiert: 2-[3-(5-Bromthiazol-2-yl)-benzyl]-6-(3-fluorphenyl)-2H-pyridazin-3-on als graue Kristalle; ESI 442,444.
2.4 Weiter wie in Beispiel 1.
   Man erhält am Ende 6-(3-Fluor-phenyl)-2-{3-[5-(1-piperidin-4-yl-1H-pyrazol-4-yl)-thiazol-2-yl]-benzyl}-2H-pyridazin-3-on als gelbe Kristalle; ESI 513; ¹H-NMR (d₆-DMSO) δ [ppm] 1.80 (m, 2H), 1.98 (m, 2H), 2.60 (m, 2H), 3.05 (m, 2H), 4.22 (m, 1H), 5.43 (s, 2H), 7.14 (d, J = 10 Hz, 1H), 7.30 (dt, J₁ = 8.5 Hz, J₂ = 2 Hz, 1H), 7.46 (m, 1H), 7.49 (t, J = 7.5 Hz, 1H), 7.55 (q, J = 7.5 Hz, 1H), 7.76 (m, 2H), 7.82 (m, 2H), 7.98 (s, 1H), 8.00 (s, 1H), 8.13 (d, J = 10 Hz, 1H), 8.23 (s, 1H).

Analog erhält man die nachstehenden Verbindungen

| Nr. | Struktur und/oder Name | |
|---|---|---|
| "A3" | 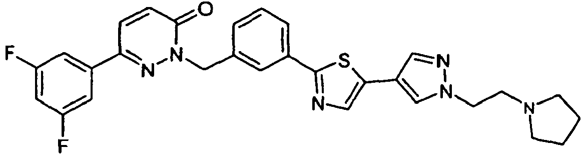 | |
| "A4" | 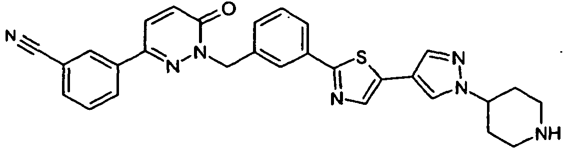 | |
| "A5" | 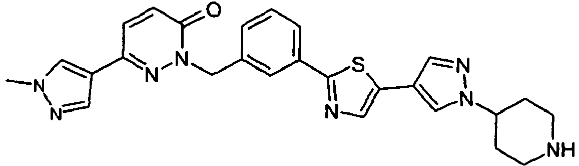 | |

### Beispiel 3

Die Herstellung von 6-(3,5-Difluor-phenyl)-2-{3-[5-(1-piperidin-4-yl-1H-pyrazol-4-yl)-oxazol-2-yl]-benzyl}-2H-pyridazin-3-on ("A6") erfolgt analog nachstehendem Schema

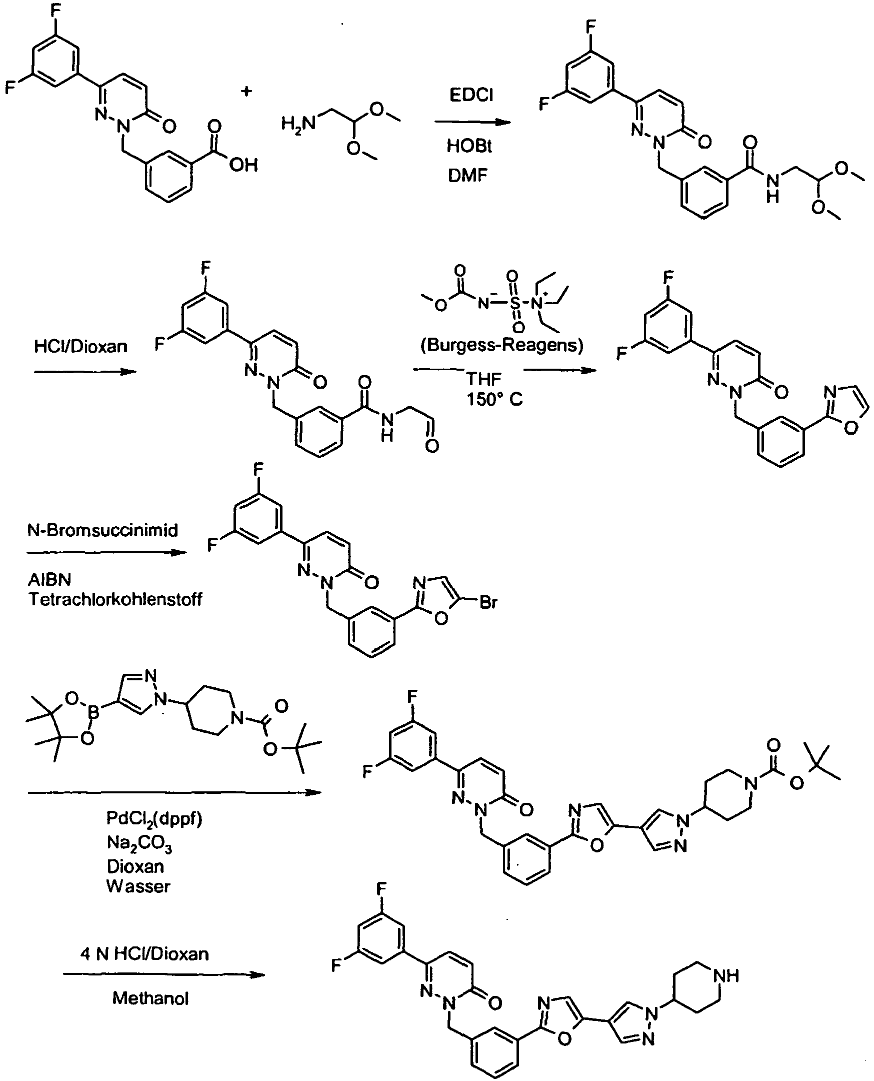

Das Ausgangsmaterial 3-[3-(3,5-Difluorphenyl)-6-oxo-6H-pyridazin-1-ylmethyl]-benzoesäure wird gemäß WO2008/017361 hergestellt. Die ersten vier Reaktionsschritte werden analog zu einer Vorschrift aus WO2005/12113 durchgeführt. Die Reaktionsschritte 4 und 5 werden analog Beispiel 1 durchgeführt.
1. Eine Lösung von 1.24 g (3.64 mmol) 3-[3-(3,5-Difluorphenyl)-6-oxo-6H-pyridazin-1-ylmethyl]-benzoesäure (hergestellt gemäß WO 2008/017361), 382 mg (3.64 mmol) Aminoacetaldehyddimethylacetal und 557 mg (3.64 mmol) 1-Hydroxybenzotriazol-hydrat (HOBt) in 14 ml DMF wird mit 906 mg (4.73 mmol) N-(3-Dimethylaminopropyl)-N'-ethylcarbodiimid-hydrochlorid (EDCI) versetzt und über Nacht bei Raumtemperatur gerührt. Das Reaktionsgemisch wird mit Wasser versetzt. Der entstandene Niederschlag wird abgesaugt, mit Wasser gewaschen und im Vakuum getrocknet. Man erhält 3-[3-(3,5-Difluorphenyl)-6-oxo-6H-pyridazin-1-ylmethyl]-N-(2,2-dimethoxy-ethyl)-benzamid als farblose Kristalle; ESI 430.
2. Eine Lösung von 1.17 g (2.73 mmol) 3-[3-(3,5-Difluorphenyl)-6-oxo-6H-pyridazin-1-ylmethyl]-N-(2,2-dimethoxy-ethyl)-benzamid in 15 ml Dioxan wird mit 4 ml 2 N wässriger Salzsäure versetzt und 20 Minuten bei einer Temperatur von 50° C gerührt. Das Reaktionsgemisch wird mit Wasser versetzt. Der entstandene Niederschlag wird abgesaugt, mit Wasser gewaschen und im Vakuum getrocknet: 3-[3-(3,5-Difluorphenyl)-6-oxo-6H-pyridazin-1-ylmethyl]-N-(2-oxo-ethyl)-benzamid als farblose Kristalle; ESI 384.
3. Eine Lösung von 1.01 g (2.64 mmol) 3-[3-(3,5-Difluorphenyl)-6-oxo-6H-pyridazin-1-ylmethyl]-N-(2-oxo-ethyl)-benzamid und 1.26 g (5.27 mmol) (Methoxycarbonylsulfamoyl)triethylammonium-betain (Burgess-Reagens) in 5.3 ml THF wird im geschlossenen Gefäß 5 Minuten bei einer Temperatur von 150° C gerührt. Das Reaktionsgemisch wird zwischen gesättiger Natriumhydrogencarbonat-Lösung und Dichlormethan verteilt. Die organische Phase wird über Natriumsulfat getrocknet und eingedampft. Der Rückstand wird an einer Kieselgelsäule mit Dichlormethan/Methanol als Laufmittel chromatographiert. Man erhält 6-(3,5-Difluorphenyl)-2-(3-oxazol-2-yl-benzyl)-2H-pyridazin-3-on als farblose Kristalle; ESI 366.
4. Eine Lösung von 184 mg (0.504 mmol) 6-(3,5-Difluorphenyl)-2-(3-oxazol-2-yl-benzyl)-2H-pyridazin-3-on, 108 mg (0.605 mmol) N-Bromsuccinimid und 6.3 mg (0.026 mmol) Benzoylperoxid in 1 ml Chlorbenzol wird 42 Stunden bei 80° C gerührt. Das Reaktionsgemisch wird eingeengt und an einer Kieselgelsäule mit Dichlormethan/Methanol als Laufmittel chromatographiert. Man erhält 2-[3-(5-Brom-oxazol-2-yl)-benzyl]-6-(3,5-difluorphenyl)-2H-pyridazin-3-on als gelbliche Kristalle; ESI 444, 446.
5. Eine unter Stickstoff gehaltene Suspension von 131 mg (0.294 mmol) 2-[3-(5-Brom-oxazol-2-yl)-benzyl]-6-(3,5-difluorphenyl)-2H-pyridazin-3-on, 144 mg (0.382 mmol) 4-[4-(4,4,5,5-Tetramethyl-[1,3,2]dioxaborolan-2-yl)-pyrazol-1-yl]-piperidin-1-carbonsäure-tert-butylester und 135 mg (0.382 mmol) Trikaliumphosphat-Trihydrat in 1.2 ml 1,2-Dimethoxyethan wird mit 21 mg (0.029 mmol) Bis-triphenylphosphin)-palladium(II)-chlorid, einem Tropfen Triethylamin und 200 µl DMF versetzt und 18 Stunden bei 80° C gerührt. Das Reaktionsgemisch wird mit THF verdünnt und abgesaugt. Das Filtrat wird zweimal mit gesättigter Natriumchlorid-Lösung gewaschen, über Natriumsulfat getrocknet und eingedampft. Der Rückstand wird an einer Kieselgelsäule mit Dichlormethan/Methanol chromatographiert. Man erhält 4-[4-(2-{3-[3-(3,5-Difluorphenyl)-6-oxo-6H-pyridazin-1-ylmethyl]-phenyl}-oxazol-5-yl)-pyrazol-1-yl]-piperidin-1-carbonsäure-tert-butylester als gelbliche Kristalle; ESI 615.
6. Eine Lösung von 90.4 mg (0.147 mmol) 4-[4-(2-{3-[3-(3,5-Difluorphenyl)-6-oxo-6H-pyridazin-1-ylmethyl]-phenyl}-oxazol-5-yl)-pyrazol-1-yl]-piperidin-1-carbonsäure-tert-butylester in einem Gemisch von 0.22 ml 4 N Chlorwasserstoff in Dioxan und 0.23 ml Methanol wird 16 Stunden bei Raumtemperatur gerührt. Die Lösung wird eingedampft und im Vakuum getrocknet. Man erhält 6-(3,5-Difluorphenyl)-2-{3-[5-(1-piperidin-4-yl-1H-pyrazol-4-yl)-oxazol-2-yl]-benzyl}-2H-pyridazin-3-on Hydrochlorid als farblose Kristalle; ESI 515.

¹H-NMR (d₆-DMSO): δ [ppm] = 2.22 (m, 4H), 3.09 (m, 2H), 3.40 (m, 2H), 4.57 (m, 1H), 5.44 (s, 2H), 7.15 (d, J = 9.8 Hz, 1H), 7.36 (tt, J₁ = 9.1 Hz, J₂ = 2.3 Hz, 1H), 7.43 (s, 1H), 7.51 (d, J = 7.5 Hz, 1H), 7.54 (t, J =7.5 Hz, 1H), 7.67 (m, 2H), 7.93 (s, 1H), 7.96 (d, J = 7.3 Hz, 1H), 8.06 (s, 1H), 8.17 (d, J = 10 Hz, 1H), 8.29 (s, 1H), 8.96 (bs, 1H, NH⁺), 9.18 (bs, 1H, NH⁺).

### Beispiel 4

Die Herstellung von 6-(3-Cyan-phenyl)-2-{3-[5-(1-piperidin-4-yl-1H-pyrazol-4-yl)-oxazol-2-yl]-benzyl}-2H-pyridazin-3-on ("A7") erfolgt analog nachstehendem Schema

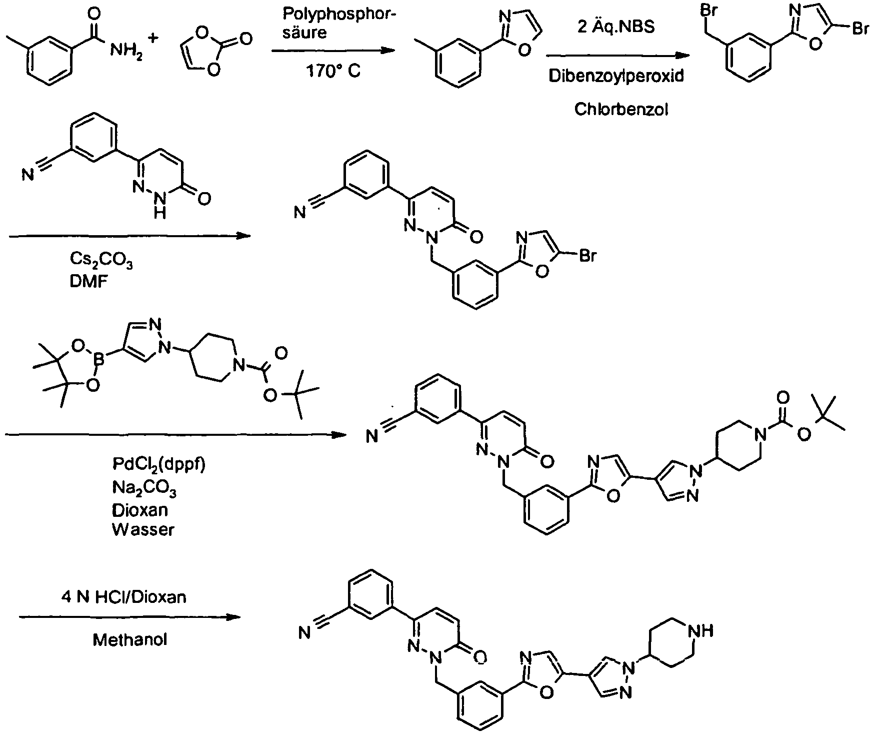

Der erste Reaktionschritt wird analog zu US2003/220272 durchgeführt, Reaktionsschritte 3, 4 und 5 analog zu Beispiel 2.

### Beispiel 5

Die Herstellung von 6-(3,5-Difluor-phenyl)-2-{3-[5-(1-methyl-1H-pyrazol-4-yl)-[1,2,4]oxadiazol-3-yl]-benzyl}-2H-pyridazin-3-on ("A8") erfolgt analog nachstehendem Schema

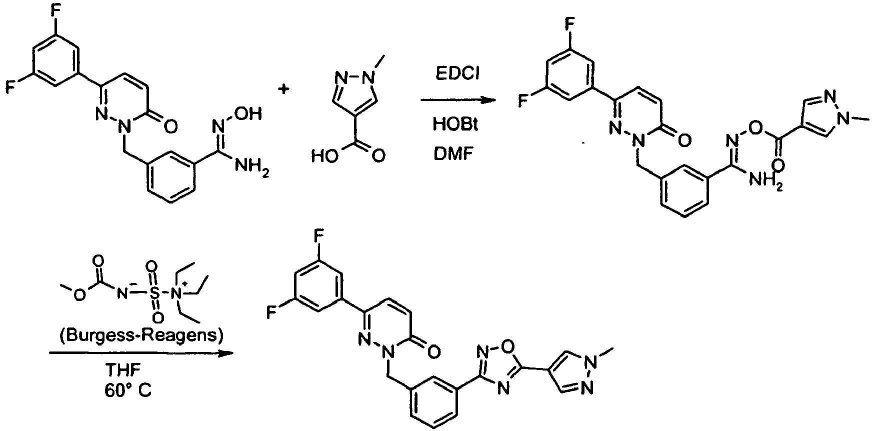

"A8": ¹H-NMR (d₆-DMSO): δ [ppm] = 3.97 (s, 3H), 5.45 (s, 2H), 7.15 (d, J = 9.5 Hz, 1H), 7.36 (tt, J₁ = 9.2 Hz, J₂ = 2 Hz, 1H), 7.57 (t, J = 7.6 Hz, 1H), 7.62 (d, J = 7.5 Hz, 1H), 7.66 (m, 2H), 7.98 (d, J = 7.6 Hz, 1H), 8.12 (s, 1H), 8.15 (d, J = 9.5 Hz, 1H), 8.16 (s, 1H), 8.68 (s, 1H).

Das Ausgangsmaterial 3-[3-(3,5-Difluorphenyl)-6-oxo-6H-pyridazin-1-ylmethyl]-N-hydroxy-benzamidin wird gemäß WO2008/017361 hergestellt.

Analog werden die nachstehenden Verbindungen erhalten

6-(3,5-Difluor-phenyl)-2-[3-(5-furan-2-yl-[1,2,4]oxadiazol-3-yl)-benzyl]-2H-pyridazin-3-on ("A9")

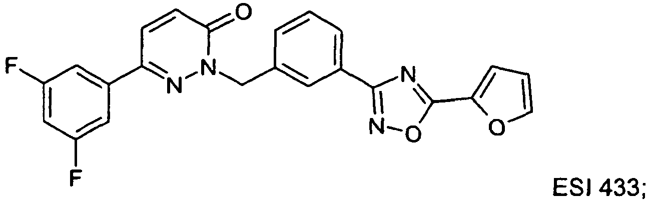

6-(3,5-Difluor-phenyl)-2-{3-[5-(6-methyl-pyridin-3-yl)-[1,2,4joxadiazol-3-yl]-benzyl}-2H-pyridazin-3-on ("A10")

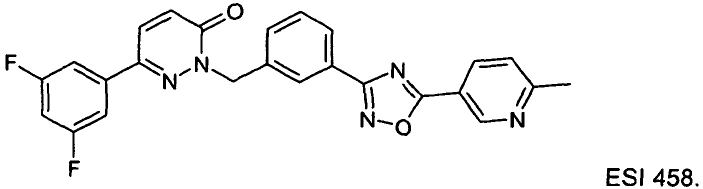

### Pharmakologische Daten

### Met-Kinase-Inhibierung

**Tabelle 1**

| Verbindung Nr. | IC₅₀ (Enzym) | IC₅₀ (Zelle) |
|---|---|---|
| "A1" | A | A |
| "A2" | A | A |
| "A6" | A | A |
| "A8" | A | A |
| "A9" | | B |
| "A10" | A | A |

| | | |
|---|---|---|
| IC₅₀: 1 nM - 0,1 µM = A; 0,1 µM-10µM=B; > 10 µM = C | | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 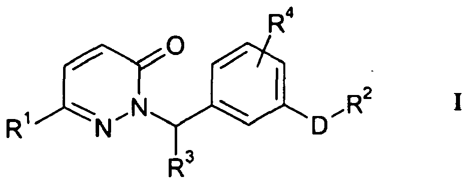 worin
D einen fünfgliedrigen ungesättigten oder aromatischen Heterocyclus mit 1 bis 3 N-, O- und/oder S-Atomen, der unsubstituiert oder ein-, zwei- oder dreifach durch Hal und/oder A substituiert sein kann,
R¹ Ar oder Het,
R² einen ungesättigten oder aromatischen fünf- oder sechsgliedrigen Heterocyclus mit 1 bis 4 N-, O- und/oder S-Atomen, der unsubstituiert oder ein- oder zweifach durch Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO(C(R³)₂]ₙ-Het¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA und/oder =O (Carbonylsauerstoff) substituiert sein kann,
R³ H oder A',
R⁴ H, A oder Hal,
A unverzweigtes oder verzweigtes Alkyl mit 1-10 C-Atomen, worin 1-7 H-Atome durch OH, F, Cl und/oder Br ersetzt sein können, und/oder worin eine oder zwei CH₂-Gruppen durch O, NH, S, SO, SO₂ und/oder CH=CH-Gruppen ersetzt sein können, oder
A' cyclisches Alkyl mit 3-7 C-Atomen, worin 1-7 H-Atome durch OH, F, Cl und/oder Br ersetzt sein können, unverzweigtes oder verzweigtes Alkyl mit 1-6 C-Atomen, worin 1-5 H-Atome durch F ersetzt sein können,
Ar unsubstituiertes oder ein-, zwei- oder dreifach durch Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, [C(R³)₂]ₙN(R³)₂, [C(R³)₂]ₙHet¹, O[C(R³)₂]ₚ-N(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂ und/oder OCONH[C(R³)₂]ₙHet¹ substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, N(R³)₂, SR³, NO₂, CN, COOR³, CON(R³)₂, NR³COA, NR³SO₂A, SO₂N(R³)₂, S(O)ₘA, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙ₋ Het¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA und/oder =O (Carbonylsauerstoff) (Carbonylsauerstoff) substituiert sein kann, Het¹ einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
Hal F, Cl, Br oder I,
m 0, 1 oder 2,
n 0, 1, 2, 3 oder 4,
p 2, 3, 4, 5, oder 6,
bedeuten,
sowie ihre pharmazeutisch verwendbaren Salze, Tautomere und Stereoisomere, einschließlich deren Mischungen in allen Verhältnissen.

2. Verbindungen nach Anspruch 1, worin
D Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
bedeutet,
sowie ihre pharmazeutisch verwendbaren Salze, Tautomere und Stereoisomere, einschließlich deren Mischungen in allen Verhältnissen.

3. Verbindungen nach Anspruch 1 oder 2, worin
R² einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl,
bedeutet, sowie ihre pharmazeutisch verwendbaren Salze, Tautomere und Stereoisomere, einschließlich deren Mischungen in allen Verhältnissen.

4. Verbindungen nach einem oder mehreren der Ansprüche 1-3, worin
R³ H oder Methyl bedeutet,
sowie ihre pharmazeutisch verwendbaren Salze, Tautomere und Stereoisomere, einschließlich deren Mischungen in allen Verhältnissen.

5. Verbindungen nach einem oder mehreren der Ansprüche 1-4, worin
R⁴ H bedeutet,
sowie ihre pharmazeutisch verwendbaren Salze, Tautomere und Stereoisomere, einschließlich deren Mischungen in allen Verhältnissen.

6. Verbindungen nach einem oder mehreren der Ansprüche 1-5, worin
Ar ein-, zwei-oder dreifach durch Hal und/oder CN substituiertes Phenyl
bedeutet,
sowie ihre pharmazeutisch verwendbaren Salze, Tautomere und Stereoisomere, einschließlich deren Mischungen in allen Verhältnissen.

7. Verbindungen nach einem oder mehreren der Ansprüche 1-6, worin
A unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
bedeutet, sowie ihre pharmazeutisch verwendbaren Salze, Tautomere und Stereoisomere, einschließlich deren Mischungen in allen Verhältnissen.

8. Verbindungen nach einem oder mehreren der Ansprüche 1-7, worin
Het einen einkernigen aromatischen Heterocyclus mit 1 bis 4 N-, O- und/oder S-Atomen, der unsubstituiert oder ein- oder zweifach durch A substituiert sein kann,
bedeutet,
sowie ihre pharmazeutisch verwendbaren Salze, Tautomere und Stereoisomere, einschließlich deren Mischungen in allen Verhältnissen.

9. Verbindungen nach einem oder mehreren der Ansprüche 1-8, worin
Het Pyrazolyl, Pyridinyl, Pyrimidinyl, Furyl, Thienyl, Oxazolyl, Oxadiazolyl, Imidazolyl, Pyrrolyl oder Isoxazolyl, wobei die Reste auch ein- oder zweifach durch A substituiert sein können,
bedeutet,
sowie ihre pharmazeutisch verwendbaren Salze, Tautomere und Stereoisomere, einschließlich deren Mischungen in allen Verhältnissen.

10. Verbindungen nach einem oder mehreren der Ansprüche 1-9, worin
Het¹ Piperidinyl, Pyrrolidinyl, Morpholinyl, Piperazinyl, Oxazolidinyl oder Imidazolidinyl, wobei die Reste auch ein- oder zweifach durch =O und/oder A substituiert sein können,
bedeutet,
sowie ihre pharmazeutisch verwendbaren Salze, Tautomere und Stereoisomere, einschließlich deren Mischungen in allen Verhältnissen.

11. Verbindungen nach einem oder mehreren der Ansprüche 1-10, worin
D Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
R¹ Ar oder Het,
R² einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl,
R³ H oder Methyl,
R⁴ H,
A unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
Ar ein-, zwei-oder dreifach durch Hal und/oder CN substituiertes Phenyl,
Het einen einkernigen aromatischen Heterocyclus mit 1 bis 4 N-, O- und/oder S-Atomen, der unsubstituiert oder ein- oder zweifach durch A substituiert sein kann,
Het¹ einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
Hal F, Cl, Br oder I,
n 0, 1, 2, 3 oder 4,
bedeuten,
sowie ihre pharmazeutisch verwendbaren Salze, Tautomere und Stereoisomere, einschließlich deren Mischungen in allen Verhältnissen.

12. Verbindungen nach einem oder mehreren der Ansprüche 1-11, worin
D Thiazol-diyl, Thiophen-diyl, Furan-diyl, Pyrrol-diyl, Oxazol-diyl, Isoxazol-diyl, Oxadiazol-diyl, Pyrazol-diyl, Imidazol-diyl, Thiadiazol-diyl, Pyridazin-diyl, Pyrazin-diyl, Pyridin-diyl oder Pyrimidin-diyl,
R¹ Ar oder Het,
R² einfach durch A oder [C(R³)₂]ₙHet¹ substituiertes Pyrazolyl, Pyridinyl, Pyrimidinyl, Furyl, Thienyl, Oxazolyl, Oxadiazolyl, Imidazolyl, Pyrrolyl oder Isoxazolyl,
R³ H oder Methyl,
R⁴ H,
A unverzweigtes oder verzweigtes Alkyl mit 1-6 C-Atomen, worin 1-5 H-Atome durch F ersetzt sein können, und/oder worin eine oder zwei CH₂-Gruppen durch O ersetzt sein können,
Ar ein-, zwei-oder dreifach durch Hal und/oder CN substituiertes Phenyl,
Het Pyrazolyl, Pyridinyl, Pyrimidinyl, Furyl, Thienyl, Oxazolyl, Oxadiazolyl, Imidazolyl, Pyrrolyl oder Isoxazolyl, wobei die Reste auch ein- oder zweifach durch A substituiert sein können,
Het¹ Piperidinyl, Pyrrolidinyl, Morpholinyl, Piperazinyl, Oxazolidinyl oder Imidazolidinyl, wobei die Reste auch ein- oder zweifach durch =O und/oder A substituiert sein können,
Hal F, Cl, Br oder I,
n 0, 1, 2, 3 oder 4,
bedeuten, sowie ihre pharmazeutisch verwendbaren Salze, Tautomere und Stereoisomere, einschließlich deren Mischungen in allen Verhältnissen.

13. Verbindungen nach Anspruch 1, ausgewählt aus der Gruppe
| Nr. | Struktur und/oder Name |
|---|---|
| "A1" | 6-(3,5-Difluor-phenyl)-2-{3-[5-(1-piperidin-4-yl-1H-pyrazol-4-yl)-thiazol-2-yl]-benzyl}-2H-pyridazin-3-on |
| "A2" | 6-(3-Fluor-phenyl)-2-{3-[5-(1-piperidin-4-yl-1H-pyrazol-4-yl)-thiazol-2-yl]-benzyl}-2H-pyridazin-3-on |
| "A3" | 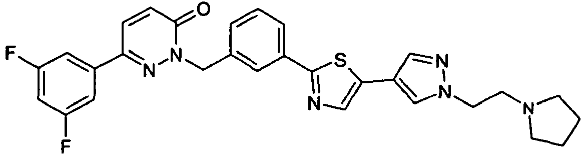 |
| "A4" | 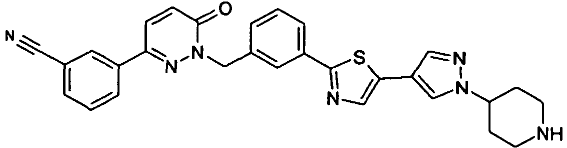 |
| "A5" | 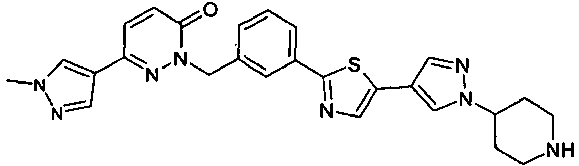 |
| "A6" | 3-(3,5-Difluor-phenyl)-2-{3-[5-(1-piperidin-4-yl-1H-pyrazol-4-yl)-oxazol-2-yl]-benzyl}-2H-pyridazin-3-on |
| "A7" | 6-(3-Cyan-phenyl)-2-{3-[5-(1-piperidin-4-yl-1H-pyrazol-4-yl)-oxazol-2-yl]-benzyl}-2H-pyridazin-3-on |
| "A8" | 6-(3,5-Difluor-phenyl)-2-{3-[5-(1-methyl-1H-pyrazol-4-yl)-[1,2,4]oxadiazol-3-yl]-benzyl}-2H-pyridazin-3-on |
| "A9" | 6-(3,5-Difluor-phenyl)-2-[3-(5-furan-2-yl-[1,2,4]oxadiazol-3-yl)-benzyl]-2H-pyridazin-3-on |
| "A10" | 6-(3,5-Difluor-phenyl)-2-{3-[5-(6-methyl-pyridin-3-yl)-[1,2,4]oxadiazol-3-yl]-benzyl}-2H-pyridazin-3-on |
sowie ihre pharmazeutisch verwendbaren Salze, Tautomere und Stereoisomere, einschließlich deren Mischungen in allen Verhältnissen.

14. Verfahren zur Herstellung von Verbindungen der Formel I nach den Ansprüchen 1-13 sowie ihrer pharmazeutisch verwendbaren Salze, Tautomeren und Stereoisomeren, **dadurch gekennzeichnet, daß** man
a) eine Verbindung der Formel II 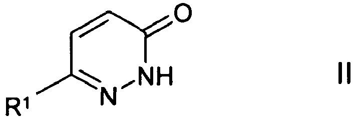
worin R¹ die in Anspruch 1 angegebene Bedeutung hat,
mit einer Verbindung der Formel III 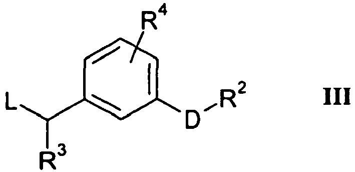
worin D, R², R³ und R⁴ die in Anspruch 1 angegebenen Bedeutungen haben und
L Cl, Br, I oder eine freie oder reaktionsfähig funktionell abgewandelte OH-Gruppe bedeutet,
umsetzt,
und/oder
eine Base oder Säure der Formel I in eines ihrer Salze umwandelt.

15. Arzneimittel, enthaltend mindestens eine Verbindung der Formel I nach Anspruch 1-13 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

16. Verwendung von Verbindungen nach Anspruch 1-13 sowie ihrer pharmazeutisch verwendbaren Salze, Tautomeren und Stereoisomeren, einschließlich deren Mischungen in allen Verhältnissen,
zur Herstellung eines Arzneimittels zur Behandlung von Krankheiten, wobei die zu behandelnde Krankheit ein fester Tumor oder ein Tumor des Blut- und Immunsystems ist.

17. Arzneimittel enthaltend mindestens eine Verbindung der Formel I gemäß einem oder mehreren der Ansprüche 1 bis 13 und/oder ihre pharmazeutisch verwendbaren Salze und Stereoisomere, einschließlich deren Mischungen in allen Verhältnissen, und mindestens einen weiteren Arzneimittelwirkstoff.

18. Set (Kit), bestehend aus getrennten Packungen von
(a) einer wirksamen Menge an einer Verbindung der Formel I gemäß einem oder mehreren der Ansprüche 1 bis 13, und/oder ihrer pharmazeutisch verwendbaren Salze und Stereoisomere, einschließlich deren Mischungen in allen Verhältnissen, und
(b) einer wirksamen Menge eines weiteren Arzneimittelswirkstoffs.

## Claims

1. Compounds of the formula I 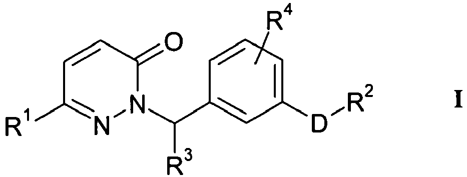 in which
D denotes a five-membered unsaturated or aromatic heterocycle having 1 to 3 N, O and/or S atoms, which may be unsubstituted or mono-, di- or trisubstituted by Hal and/or A,
R¹ denotes Ar or Het,
R² denotes an unsaturated or aromatic five- or six-membered heterocycle having 1 to 4 N, O and/or S atoms, which may be unsubstituted or mono- or disubstituted by Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA and/or =O (carbonyl oxygen),
R³ denotes H or A',
R⁴ denotes H, A or Hal,
A denotes unbranched or branched alkyl having 1-10 C atoms, in which 1-7 H atoms may be replaced by OH, F, Cl and/or Br, and/or in which one or two CH₂ groups may be replaced by O, NH, S, SO, SO₂ and/or CH=CH groups, or cyclic alkyl having 3-7 C atoms, in which 1-7 H atoms
A' may be replaced by OH, F, Cl and/or Br, denotes unbranched or branched alkyl having 1-6 C atoms, in which 1-5 H atoms may be replaced by F,
Ar denotes phenyl, naphthyl or biphenyl, each of which is unsubstituted or mono-, di- or trisubstituted by Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂ and/or OCONH[C(R³)₂]ₙHet¹,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, N(R³)₂, SR³, NO₂, CN, COOR³, CON(R³)₂, NR³COA, NR³SO₂A, SO₂N(R³)₂, S(O)ₘA, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA and/or =O (carbonyl oxygen),
Het¹ denotes a monocyclic saturated heterocycle having 1 to 2 N and/or O atoms, which may be mono- or disubstituted by A, OA, OH, Hal and/or =O (carbonyl oxygen),
Hal denotes F, Cl, Br or I,
m denotes 0, 1 or 2,
n denotes 0, 1, 2, 3 or 4,
p denotes 2, 3, 4, 5 or 6,
and pharmaceutically usable salts, tautomers and stereoisomers thereof, including mixtures thereof in all ratios.

2. Compounds according to Claim 1 in which
D denotes thiazolediyl, thiophenediyl, furandiyl, pyrrolediyl, oxazolediyl, isoxazolediyl, oxadiazolediyl, pyrazolediyl, imidazolediyl, thiadiazolediyl, pyridazinediyl, pyrazinediyl, pyridinediyl or pyrimidinediyl,
and pharmaceutically usable salts, tautomers and stereoisomers thereof, including mixtures thereof in all ratios.

3. Compounds according to Claim 1 or 2 in which
R² denotes pyrazolyl, pyridinyl, pyrimidinyl, furyl, thienyl, oxazolyl, oxadiazolyl, imidazolyl, pyrrolyl or isoxazolyl, each of which is monosubstituted by A or [C(R³)₂]ₙHet¹,
and pharmaceutically usable salts, tautomers and stereoisomers thereof, including mixtures thereof in all ratios.

4. Compounds according to one or more of Claims 1-3 in which
R³ denotes H or methyl,
and pharmaceutically usable salts, tautomers and stereoisomers thereof, including mixtures thereof in all ratios.

5. Compounds according to one or more of Claims 1-4 in which
R⁴ denotes H,
and pharmaceutically usable salts, tautomers and stereoisomers thereof, including mixtures thereof in all ratios.

6. Compounds according to one or more of Claims 1-5 in which
Ar denotes phenyl which is mono-, di- or trisubstituted by Hal and/or CN,
and pharmaceutically usable salts, tautomers and stereoisomers thereof, including mixtures thereof in all ratios.

7. Compounds according to one or more of Claims 1-6 in which
A denotes unbranched or branched alkyl having 1-6 C atoms, in which 1-5 H atoms may be replaced by F, and/or in which one or two CH₂ groups may be replaced by O,
and pharmaceutically usable salts, tautomers and stereoisomers thereof, including mixtures thereof in all ratios.

8. Compounds according to one or more of Claims 1-7 in which
Het denotes a monocyclic aromatic heterocycle having 1 to 4 N, O and/or S atoms, which may be unsubstituted or mono- or disubstituted by A,
and pharmaceutically usable salts, tautomers and stereoisomers thereof, including mixtures thereof in all ratios.

9. Compounds according to one or more of Claims 1-8 in which
Het denotes pyrazolyl, pyridinyl, pyrimidinyl, furyl, thienyl, oxazolyl, oxadiazolyl, imidazolyl, pyrrolyl or isoxazolyl, where the radicals may also be mono- or disubstituted by A,
and pharmaceutically usable salts, tautomers and stereoisomers thereof, including mixtures thereof in all ratios.

10. Compounds according to one or more of Claims 1-9 in which
Het¹ denotes piperidinyl, pyrrolidinyl, morpholinyl, piperazinyl, oxazolidinyl or imidazolidinyl, where the radicals may also be mono- or disubstituted by =O and/or A,
and pharmaceutically usable salts, tautomers and stereoisomers thereof, including mixtures thereof in all ratios.

11. Compounds according to one or more of Claims 1-10 in which
D denotes thiazolediyl, thiophenediyl, furandiyl, pyrrolediyl, oxazolediyl, isoxazolediyl, oxadiazolediyl, pyrazolediyl, imidazolediyl, thiadiazolediyl, pyridazinediyl, pyrazinediyl, pyridinediyl or pyrimidinediyl,
R¹ denotes Ar or Het,
R² denotes pyrazolyl, pyridinyl, pyrimidinyl, furyl, thienyl, oxazolyl, oxadiazolyl, imidazolyl, pyrrolyl or isoxazolyl, each of which is monosubstituted by A or [C(R³)₂]ₙHet¹,
R³ denotes H or methyl,
R⁴ denotes H,
A denotes unbranched or branched alkyl having 1-6 C atoms, in which 1-5 H atoms may be replaced by F, and/or in which one or two CH₂ groups may be replaced by O,
Ar denotes phenyl which is mono-, di- or trisubstituted by Hal and/or CN,
Het denotes a monocyclic aromatic heterocycle having 1 to 4 N, O and/or S atoms, which may be unsubstituted or mono- or disubstituted by A,
Het¹ denotes a monocyclic saturated heterocycle having 1 to 2 N and/or O atoms, which may be mono- or disubstituted by A, OA, OH, Hal and/or =O (carbonyl oxygen),
Hal denotes F, Cl, Br or I,
n denotes 0, 1, 2, 3 or 4,
and pharmaceutically usable salts, tautomers and stereoisomers thereof, including mixtures thereof in all ratios.

12. Compounds according to one or more of Claims 1-11 in which
D denotes thiazolediyl, thiophenediyl, furandiyl, pyrrolediyl, oxazolediyl, isoxazolediyl, oxadiazolediyl, pyrazolediyl, imidazolediyl, thiadiazolediyl, pyridazinediyl, pyrazinediyl, pyridinediyl or pyrimidinediyl,
R¹ denotes Ar or Het,
R² denotes pyrazolyl, pyridinyl, pyrimidinyl, furyl, thienyl, oxazolyl, oxadiazolyl, imidazolyl, pyrrolyl or isoxazolyl, each of which is monosubstituted by A or [C(R³)₂]ₙHet¹,
R³ denotes H or methyl,
R⁴ denotes H,
A denotes unbranched or branched alkyl having 1-6 C atoms, in which 1-5 H atoms may be replaced by F, and/or in which one or two CH₂ groups may be replaced by O,
Ar denotes phenyl which is mono-, di- or trisubstituted by Hal and/or CN,
Het denotes pyrazolyl, pyridinyl, pyrimidinyl, furyl, thienyl, oxazolyl, oxadiazolyl, imidazolyl, pyrrolyl or isoxazolyl, where the radicals may also be mono- or disubstituted by A,
Het¹ denotes piperidinyl, pyrrolidinyl, morpholinyl, piperazinyl, oxazolidinyl or imidazolidinyl, where the radicals may also be mono- or disubstituted by =O and/or A,
Hal denotes F, Cl, Br or I,
n denotes 0, 1, 2, 3 or 4,
and pharmaceutically usable salts, tautomers and stereoisomers thereof, including mixtures thereof in all ratios.

13. Compounds according to Claim 1, selected from the group
| No. | Structure and/or name |
|---|---|
| "A1" | 6-(3,5-Difluorophenyl)-2-{3-[5-(1-piperidin-4-yl-1H-pyrazol-4-yl)thiazol-2-yl]benzyl}-2H-pyridazin-3-one |
| "A2" | 6-(3-Fluorophenyl)-2-{3-[5-(1-piperidin-4-yl-1H-pyrazol-4-yl)thiazol-2-yl]benzyl}-2H-pyridazin-3-one |
| "A3" | 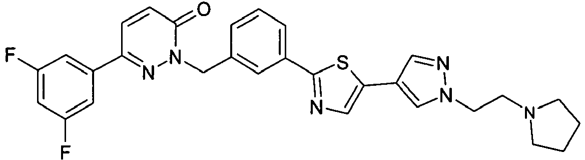 |
| "A4" | 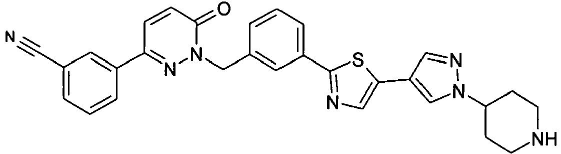 |
| "A5" | 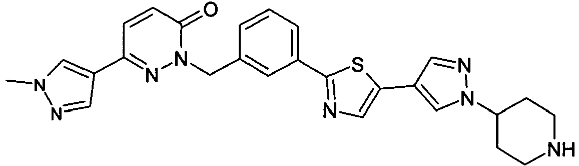 |
| "A6" | 6-(3,5-Difluorophenyl)-2-{3-[5-(1-piperidin-4-yl-1H-pyrazol-4-yl)oxazol-2-yl]benzyl}-2H-pyridazin-3-one |
| "A7" | 3-(3-Cyanophenyl)-2-{3-[5-(1-piperidin-4-yl-1H-pyrazol-4-yl)-oxazol-2-yl]benzyl}-2H-pyridazin-3-one |
| "A8" | 6-(3,5-Difluorophenyl)-2-{3-[5-(1-methyl-H-pyrazol-4-yl)-1,2,4-oxadiazol-3-yl]benzyl}-2H-pyridazin-3-one |
| "A9" | 6-(3,5-Difluorophenyl)-2-[3-(5-furan-2-yl-1,2,4-oxadiazol-3-yl)benzyl]-2H-pyridazin-3-one |
| "A10" | 6-(3,5-Difluorophenyl)-2-{3-[5-(6-methylpyridin-3-yl)-1,2,4-oxadiazol-3-yl]benzyl}-2H-pyridazin-3-one |
and pharmaceutically usable salts, tautomers and stereoisomers thereof, including mixtures thereof in all ratios.

14. Process for the preparation of compounds of the formula I according to Claims 1-13 and pharmaceutically usable salts, tautomers and stereoisomers thereof, **characterised in that**
a) a compound of the formula II 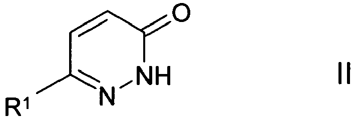
in which R¹ has the meaning indicated in Claim 1,
is reacted with a compound of the formula III 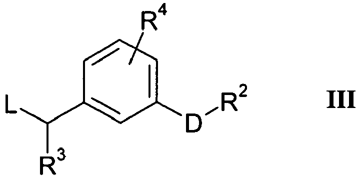
in which D, R², R³ and R⁴ have the meanings indicated in Claim 1 and
L denotes Cl, Br, I or a free or reactively functionally modified OH group,
and/or
a base or acid of the formula I is converted into one of its salts.

15. Medicaments comprising at least one compound of the formula I according to Claims 1-13 and/or pharmaceutically usable salts, tautomers and stereoisomers thereof, including mixtures thereof in all ratios, and optionally excipients and/or adjuvants.

16. Use of compounds according to Claims 1-13 and pharmaceutically usable salts, tautomers and stereoisomers thereof, including mixtures thereof in all ratios,
for the preparation of a medicament for the treatment of diseases, where the disease to be treated is a solid tumour or a tumour of the blood and immune system.

17. Medicaments comprising at least one compound of the formula I according to one or more of Claims 1 to 13 and/or pharmaceutically usable salts and stereoisomers thereof, including mixtures thereof in all ratios, and at least one further medicament active compound.

18. Set (kit) consisting of separate packs of
(a) an effective amount of a compound of the formula I according to one or more of Claims 1 to 13, and/or pharmaceutically usable salts and stereoisomers thereof, including mixtures thereof in all ratios,
and
(b) an effective amount of a further medicament active compound.

## Revendications

1. Composés de la formule I 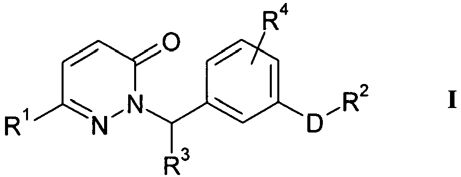 dans laquelle
D représente un hétérocycle non saturé ou aromatique à cinq éléments comportant de 1 à 3 atomes de N, de O et/ou de S, lequel peut être non substitué ou mono-, di- ou trisubstitué par Hal et/ou A,
R¹ représente Ar ou Het,
R² représente un hétérocycle non saturé ou aromatique à cinq ou six or six éléments comportant de 1 à 4 atomes de N, de O et/ou de S, lequel peut être non substitué ou mono- ou disubstitué par Hal, A, OH, OA, N(R³)₂, SR³, NO₂, CN, COOR³, CON(R³)₂, NR³COA, NR ³SO₂A, SO₂N(R³)₂, S(O)ₘA, CO-Het¹, Het¹, [C(R³)₂]ₙN(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA et/ou =O (oxygène carbonyle),
R³ représente H ou A',
R⁴ représente H, A ou Hal,
A représente alkyle non ramifié ou ramifié comportant 1-10 atomes de C, où 1-7 atomes de H peut/peuvent être remplacé(s) par OH, F, CI et/ou Br, et/ou où un ou deux groupes CH₂ peut/peuvent être remplacé(s) par O, NH, S, SO, SO₂ et/ou des groupes CH=CH, ou alkyle cyclique comportant 3-7 atomes de C, où 1-7 atomes de H peut/peuvent être remplacé(s) par OH, F, CI et/ou Br,
A' représente alkyle non ramifié ou ramifié comportant 1-6 atomes de C, où 1-5 atomes de H peut/peuvent être remplacé(s) par F,
Ar représente phényle, naphthyle ou biphényle dont chacun est non substitué ou mono-, di- ou trisubstitué par Hal, A, OH, OA, N(R³)₂, SR³, NO₂, CN, COOR³, CON(R³)₂, NR³COA, NR³SO₂A, SO₂N(R³)₂, S(O)ₘA, CO-Het¹, [C(R³)₂]ₙN(R³)₂, [C(R³)₂]ₙHet¹, O[C(R³)₂]ₚ-N(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂ et/ou OCONH[C(R³)₂]ₙHet¹,
Het représente un hétérocycle mono-, bi- ou tricyclique saturé, non saturé ou aromatique comportant de 1 à 4 atomes de N, de O et/ou de S, lequel peut être non substitué ou mono-, di- ou trisubstitué par Hal, A, OH, OA, N(R³)₂, SR³, NO₂, CN, COOR³, CON(R³)₂, NR³COA, NR³SO₂A, SO₂N(R³)₂, S(O)ₘA, Het¹, [C(R³)₂]ₙ-N(R³)₂, [C(R³)₂]ₙHet¹, O[C(R³)₂]ₚN(R³)₂, O[C(R³)₂]ₙHet¹, NHCOOA, NHCON(R³)₂, NHCOO[C(R³)₂]ₚN(R³)₂, NHCOO[C(R³)₂]ₙHet¹, NHCONH[C(R³)₂]ₚN(R³)₂, NHCONH[C(R³)₂]ₙHet¹, OCONH[C(R³)₂]ₚN(R³)₂, OCONH[C(R³)₂]ₙHet¹, CO-Het¹, CHO, COA, =S, =NH, =NA et/ou =O (oxygène carbonyle),
Het¹ représente un hétérocycle saturé monocyclique comportant de 1 à 2 atomes de N et/ou de O, lequel peut être mono- ou disubstitué par A, OA, OH, Hal et/ou =O (oxygène carbonyle),
Hal représente F, CI, Br ou I,
m représente 0, 1 ou 2,
n représente 0, 1, 2, 3 ou 4,
p représente 2, 3, 4, 5 ou 6,
et leurs sels, tautomères et stéréoisomères pharmaceutiquement utilisables, y compris des mélanges afférents selon toutes proportions.

2. Composés selon la revendication 1 dans lesquels
D représente thiazolediyle, thiophènediyle, furandiyle, pyrrolediyle, oxazolediyle, isoxazolediyle, oxadiazolediyle, pyrazolediyle, imidazolediyle, thiadiazolediyle, pyridazinediyle, pyrazinediyle, pyridinediyle ou pyrimidinediyle,
et leurs sels, tautomères et stéréoisomères pharmaceutiquement utilisables, y compris des mélanges afférents selon toutes proportions.

3. Composés selon la revendication 1 ou 2 dans lesquels
R² représente pyrazolyle, pyridinyle, pyrimidinyle, furyle, thiényle, oxazolyle, oxadiazolyle, imidazolyle, pyrrolyle ou isoxazolyle dont chacun est monosubstitué par A ou [C(R³)₂]ₙHet¹,
et leurs sels, tautomères et stéréoisomères pharmaceutiquement utilisables, y compris des mélanges afférents selon toutes proportions.

4. Composés selon une ou plusieurs des revendications 1-3 dans lesquels
R³ représente H ou méthyle,
et leurs sels, tautomères et stéréoisomères pharmaceutiquement utilisables, y compris des mélanges afférents selon toutes proportions.

5. Composés selon une ou plusieurs des revendications 1-4 dans lesquels
R⁴ représente H,
et leurs sels, tautomères et stéréoisomères pharmaceutiquement utilisables, y compris des mélanges afférents selon toutes proportions.

6. Composés selon une ou plusieurs des revendications 1-5 dans lesquels
Ar représente phényle qui est mono-, di- ou trisubstitué par Hal et/ou CN,
et leurs sels, tautomères et stéréoisomères pharmaceutiquement utilisables, y compris des mélanges afférents selon toutes proportions.

7. Composés selon une ou plusieurs des revendications 1-6 dans lesquels
A représente alkyle non ramifié ou ramifié comportant 1-6 atomes de C, où 1-5 atomes de H peut/peuvent être remplacé(s) par F, et/ou où un ou deux groupes CH₂ peut/peuvent être remplacé(s) par O,
et leurs sels, tautomères et stéréoisomères pharmaceutiquement utilisables, y compris des mélanges afférents selon toutes proportions.

8. Composés selon une ou plusieurs des revendications 1-7 dans lesquels
Het représente un hétérocycle aromatique monocyclique comportant de 1 à 4 atomes de N, de O et/ou de S, lequel peut être non substitué ou mono- ou disubstitué par A,
et leurs sels, tautomères et stéréoisomères pharmaceutiquement utilisables, y compris des mélanges afférents selon toutes proportions.

9. Composés selon une ou plusieurs des revendications 1-8 dans lesquels
Het représente pyrazolyle, pyridinyle, pyrimidinyle, furyle, thiényle, oxazolyle, oxadiazolyle, imidazolyle, pyrrolyle ou isoxazolyle, où les radicaux peuvent également être mono- ou disubstitués par A,
et leurs sels, tautomères et stéréoisomères pharmaceutiquement utilisables, y compris des mélanges afférents selon toutes proportions.

10. Composés selon une ou plusieurs des revendications 1-9 dans lesquels
Het¹ représente pipéridinyle, pyrrolidinyle, morpholinyle, pipérazinyle, oxazolidinyle ou imidazolidinyle, où les radicaux peuvent également être mono- ou disubstitués par =O et/ou A,
et leurs sels, tautomères et stéréoisomères pharmaceutiquement utilisables, y compris des mélanges afférents selon toutes proportions.

11. Composés selon une ou plusieurs des revendications 1-10 dans lesquels
D représente thiazolediyle, thiophènediyle, furandiyle, pyrrolediyle, oxazolediyle, isoxazolediyle, oxadiazolediyle, pyrazolediyle, imidazolediyle, thiadiazolediyle, pyridazinediyle, pyrazinediyle, pyridinediyle ou pyrimidinediyle,
R¹ représente Ar ou Het,
R² représente pyrazolyle, pyridinyle, pyrimidinyle, furyle, thiényle, oxazolyle, oxadiazolyle, imidazolyle, pyrrolyle ou isoxazolyle, dont chacun est monosubstitué par A ou [C(R³)₂]ₙHet¹,
R³ représente H ou méthyle,
R⁴ représente H,
A représente alkyle non ramifié ou ramifié comportant 1-6 atomes de C, où 1-5 atomes de H peut/peuvent être remplacé(s) par F, et/ou où un ou deux groupes CH₂ peut/peuvent être remplacé(s) par O,
Ar représente phényle qui est mono-, di- ou trisubstitué par Hal et/ou CN,
Het représente un hétérocycle aromatique monocyclique comportant de 1 à 4 atomes de N, de O et/ou de S, lequel peut être non substitué ou mono- ou disubstitué par A,
Het¹ représente un hétérocycle saturé monocyclique comportant de 1 à 2 atomes de N et/ou de O, lequelù peut être mono- ou disubstitué par A, OA, OH, Hal et/ou =O (oxygène carbonyle),
Hal représente F, CI, Br ou I,
n représente 0, 1, 2, 3 ou 4,
et leurs sels, tautomères et stéréoisomères pharmaceutiquement utilisables, y compris des mélanges afférents selon toutes proportions.

12. Composés selon une ou plusieurs des revendications 1-11 dans lesquels
D représente thiazolediyle, thiophènediyle, furandiyle, pyrrolediyle, oxazolediyle, isoxazolediyle, oxadiazolediyle, pyrazolediyle, imidazolediyle, thiadiazolediyle, pyridazinediyle, pyrazinediyle, pyridinediyle ou pyrimidinediyle,
R¹ représente Ar ou Het,
R² représente pyrazolyle, pyridinyle, pyrimidinyle, furyle, thiényle, oxazolyle, oxadiazolyle, imidazolyle, pyrrolyle ou isoxazolyle dont chacun est monosubstitué par A ou [C(R³)₂]ₙHet¹,
R³ représente H ou méthyle,
R⁴ représente H,
A représente alkyle non ramifié ou ramifié comportant 1-6 atomes de C, où 1-5 atomes de H peut/peuvent être remplacé(s) par F, et/ou où un ou deux groupes CH₂ peut/peuvent être remplacé(s) par O,
Ar représente phényle qui est mono-, di- ou trisubstitué par Hal et/ou CN,
Het représente pyrazolyle, pyridinyle, pyrimidinyle, furyle, thiényle, oxazolyle, oxadiazolyle, imidazolyle, pyrrolyle ou isoxazolyle, où les radicaux peuvent également être mono- ou disubstitués par A,
Het¹ représente pipéridinyle, pyrrolidinyle, morpholinyle, pipérazinyle, oxazolidinyle ou imidazolidinyle, où les radicaux peuvent également être mono- ou disubstitués par =O et/ou A,
Hal représente F, Cl, Br ou I,
n représente 0, 1, 2, 3 ou 4,
et leurs sels, tautomères et stéréoisomères pharmaceutiquement utilisables, y compris des mélanges afférents selon toutes proportions.

13. Composés selon la revendication 1, choisis parmi le groupe
| No. | Structure et/ou nom |
|---|---|
| "A1" | 6-(3,5-difluorophényl)-2-{3-[5-(1-pipéridine-4-yl-1H-pyrazol-4-yl)thiazol-2-yl]benzyl}-2H-pyridazine-3-one |
| "A2" | 6-(3-fluorophényl)-2-{3-[5-(1-pipéridine-4-yl-1H-pyrazol-4-yl)thiazol-2-yl]benzyl}-2H-pyridazine-3-one |
| "A3" | 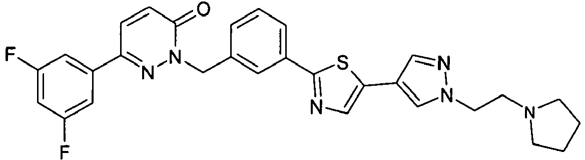 |
| "A4" | 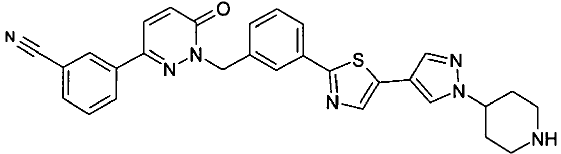 |
| "A5" | 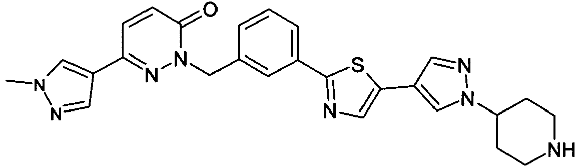 |
| "A6" | 6-(3,5-difluorophényl)-2-{3-[5-(1-pipéridine-4-yl-1H-pyrazol-4-yl)oxazol-2-yl]benzyl}-2H-pyridazine-3-one |
| "A7" | 6-(3-cyanophényl)-2-{3-[5-(1-pipéridine-4-yl-1H-pyrazol-4-yl)oxazol-2-yl]benzyl}-2H-pyridazine-3-one |
| "A8" | 6-(3,5-difluorophényl)-2-{3-[5-(1-méthyl-1H-pyrazol-4-yl)-1,2,4-oxadiazol-3-yl]benzyl}-2H-pyridazin-3-one |
| "A9" | 6-(3,5-difluorophényl)-2-[3-(5-furan-2-yl-1,2,4-oxadiazol-3-yl)benzyl]-2H-pyridazin-3-one |
| "A10" | 6-(3,5-difluorophényl)-2-{3-[5-(6-méthylpyridin-3-yl)-1,2,4-oxadiazol-3-yl]benzyl}-2H-pyridazin-3-one |
et leurs sels, tautomères et stéréoisomères pharmaceutiquement utilisables, y compris des mélanges afférents selon toutes proportions.

14. Procédé pour la préparation de composés de la formule I selon les revendications 1-13 et leurs sels, tautomères et stéréoisomères pharmaceutiquement utilisables, y compris des mélanges afférents selon toutes proportions, **caractérisé en ce que**
a) un composé de la formule II 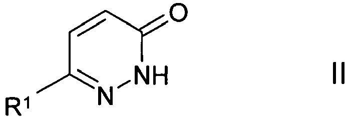
dans laquelle R¹ présente la signification indiquée selon la revendication 1,
est amené à réagir avec un composé de la formule III 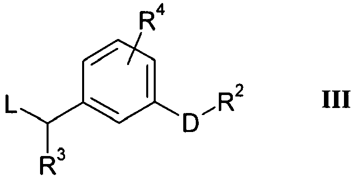
dans laquelle D, R², R³ and R⁴ présentent les significations indiquées selon la revendication 1 et
L représente Cl, Br, I ou un groupe OH libre ou modifié fonctionnellement par réaction,
et/ou
une base ou un acide de la formule I est converti(e) selon l'un de ses sels.

15. Médicaments comprenant au moins un composé de la formule I selon les revendications 1-13 et/ou leurs sels, tautomères et stéréoisomères pharmaceutiquement utilisables, y compris des mélanges afférents selon toutes proportions, et en option, des excipients et/ou adjuvants.

16. Utilisation de composés selon les revendications 1-13 et leurs sels, tautomères et stéréoisomères pharmaceutiquement utilisables, y compris des mélanges afférents selon toutes proportions,
pour la préparation d'un médicament pour le traitement de maladies, où la maladie à traiter est une tumeur solide un une tumeur du sang et du système immunitaire.

17. Médicaments comprenant au moins un composé de la formule I selon une ou plusieurs des revendications 1 à 13 et/ou leurs sels, tautomères et stéréoisomères pharmaceutiquement utilisables, y compris des mélanges afférents selon toutes proportions, et au moins un autre composé actif de médicament.

18. Ensemble (kit) constitué par des packs séparés de
(a) une quantité efficace d'un composé de la formule I selon une ou plusieurs des revendications 1 à 13, et/ou leurs sels, tautomères et stéréoisomères pharmaceutiquement utilisables, y compris des mélanges afférents selon toutes proportions,
et
(b) une quantité efficace d'un autre composé actif de médicament.
